# EUROPEAN PATENT APPLICATION

(11) **EP 4 589 005 A1**
(43) Date of publication of application: **23.07.2025**
(21) Application number: 23865583.1
(22) Date of filing: 13.09.2023
(51) Int. Cl.: C12N 5/10, C12N 15/12, C12N 15/54, A61K 35/17

(54) **T CELLS FOR TRANSPLANTATION AND PRODUCTION METHOD THEREOF**

(30) Priority: 13.09.2022 JP 2022145680
(71) Applicant: Cyto-Facto Inc., Kobe-shi, Hyogo 650-0047 (JP)
(72) Inventor: KAWAMATA, Shin, Kobe-shi, Hyogo 650-0047 (JP); YAMAMOTO, Takako, Kobe-shi, Hyogo 651-0097 (JP)
(74) Representative: Vossius & Partner Patentanwälte Rechtsanwälte mbB
(86) International application number: PCT/JP2023/033465
(87) International publication number: WO 2024/058234

(57) **Abstract**

The present invention provides a human T cell lacking human leukocyte antigen (HLA) class I molecule and containing an exogenous ST6GALNAC6 gene, which has the following characteristics (a) and/or (b):
(a) CD62L-positive and CD45RA-positive
(b) a total ATP production rate of a human T cell-containing cell population of 400 pmol/min/10⁵ cells or more and a mitochondrial spare respiratory capacity of 40 pmol/min/10⁵ cells or more, 5-6 days after the application of T cell proliferation stimulation to the cell population. According to the present invention, a low-immunogenic human T cell that can remain stably in the body for a long period of time after transplantation is provided, and the development of a versatile CAR-T cell or TCR-T cell for allotransplantation becomes possible by using the human T cell.

## Description

### [Technical Field]

The present disclosure relates to human T cells that can remain in the body for a long period of time after transplantation, in particular, genetically modified human T cells with extremely low-immunogenicity, a method for producing the T cells, and medical use of the T cells.

### [Background Art]

CAR-T cell (Chimeric Antigen Receptor-T cell) therapy and TCR-T cell (T Cell Receptor-T cell) therapy are treatment methods in which T cell, which is one type of lymphocyte, is extracted from the patient's blood and genetically modified, and the genetically modified T cell is specifically bound to the surface antigen of specific cancer cells, whereby a high CTL effect is expected. However, due to the issue of graft-versus-host disease (GVHD), autologous transplantation is currently the mainstream. Therefore, these therapies are time-consuming tailored medicaments with high drug prices, and do not allow stockpiling as an off-the-shelf product or timely provision according to medical conditions. These, along with off-target (on target/off tumor) issues, are obstacles to the widespread use of these therapies.

In addition, in the case of the treatment of B-cell leukemia, CAR-T cells or TCR-T cells must remain in the recipient for at least six months to exert a long-term killer T cell (CTL) effect, in order to eradicate minute residual cancer cells. Therefore, it is also necessary to overcome technical problems that enable stable long-term retention in the body after transplantation.

As a means of immune evasion to enable universal allogeneic transplantation, attempts have long been made to produce low-immunogenic cells by deleting the human leukocyte antigen (HLA) gene. This is because HLA is recognized as the most important antigen (exogenous substance) by immunocompetent cells such as CTL and the like in allogeneic transplantation and causes rejection. With the rapid spread and development of genome editing technology and the like in recent years, genetic modification of cells can now be performed easily and accurately, and attempts to produce low-immunogenic pluripotent stem cells are progressing rapidly. For example, Universal Cell and the University of Washington have developed low-immunogenic cells that do not express HLA molecules and can avoid attacks from CTL and the like, by deleting the β2 microglobulin (B2M) gene, which is the common light chain of HLA class I molecules, and the RFXANK gene, which is a transcription factor required for the expression of HLA class II molecules in pluripotent stem cells (Patent Literatures 1 and 2).

However, cells lacking HLA are subject to attack by NK cells because HLA is an inhibitory molecule for natural killer (NK) cells. A group from the University of California has similarly eliminated the expression of HLA molecules by deleting the B2M gene and the CIITA gene, which is another transcription factor required for the expression of HLA class II molecules, and developed low-immunogenic iPS cells that overexpress CD47, which inhibits phagocytosis by macrophages and cytotoxicity by NK cells (Non Patent Literature 1). A group from Harvard University has also disclosed low-immunogenic therapeutic cells in which the B2M gene and the CIITA gene have been deleted and the HLA-G gene has been knocked in to avoid attack by NK cells (Patent Literature 3). On the other hand, a group from Kyoto University has produced human iPS cells in which only the HLA-A and HLA-B genes have been individually deleted, and the CIITA gene has also been deleted (Non Patent Literature 2).

As described above, various low-immunogenic cells have been produced by deleting or introducing various genes, including HLA genes, into pluripotent stem cells. However, as the situation stands, low-immunogenic T cells that can remain in the body stably for a long time after transplantation and serve as a platform for the production of CAR-T have not yet been obtained.

### [Citation List]

### [Patent Literature]

[Patent Literature 1]
   WO 2012/145384
[Patent Literature 2]
   WO 2013/158292
[Patent Literature 3]
   WO 2016/183041

### [Non Patent Literature]

[Non Patent Literature 1]
   Tobias Deuse et al., Nature Biotechnology, volume 37, pages 252-258, 2019
[Non Patent Literature 2]
   Huaigeng Xu et al., Cell Stem Cell, 24, 566-578, 2019

### [Summary of Invention]

### [Technical Problem]

Therefore, the present invention aims to provide a human T cell that can remain stably in the body for a long time after transplantation and a production method thereof, as well as a low-immunogenic human T cell using the human T cell, which is suitable for allotransplantation, and a production method thereof, and to provide a versatile CAR-T cell or TCR-T cell for allotransplantation, by using the low-immunogenic human T cell.

### [Solution to Problem]

The present inventors conceived an idea that the most undifferentiated human T cell fraction is expected to remain in the body for a long time after transplantation. They have succeeded in isolating and amplifying the CD62L⁺CD45RA⁺ fraction containing memory T cells from human peripheral blood, and establishing a culture system capable of maintenance culture for a long time after cryopreservation and thawing. Furthermore, in this fraction of T cells, they knocked out (KO) the B2M gene by gene editing technology, thereby eliminating the expression of all HLA class I molecules (HLA-A, B, C, E, G, F). Furthermore, they have succeeded in establishing low-immunogenic T cells that are not attacked by NK cells by forcibly expressing, in B2M KO T cells, the ST6GALNAC6 gene, which encodes the synthetic enzyme of disialosylglobopentaosylceramide (DSGb5), which specifically binds to the NK cell receptor Siglec-7 (CD328).

In addition, the present inventors succeeded in obtaining a human T cell fraction that is superior in expansion efficiency in maintenance culture and can remain in the body for a long period after transplantation, by using the amount of cellular ATP production and mitochondrial spare respiratory capacity as indicators instead of the expression of cell surface markers.

Based on these findings, the present inventors have conducted further studies and completed the present invention.

That is, the present invention provides the following.
[1] A human T cell lacking human leukocyte antigen (HLA) class I molecule and comprising an exogenous ST6GALNAC6 gene, which has the following characteristics (a) and/or (b):
   (a) CD62L-positive and CD45RA-positive
   (b) a total ATP production rate of a human T cell-containing cell population of 400 pmol/min/10⁵ cells or more and a mitochondrial spare respiratory capacity of 40 pmol/min/10⁵ cells or more, 5-6 days after the application of T cell proliferation stimulation to the cell population.
[1A] The human T cell of [1], which further lacks T cell receptor α chain constant region (TRAC) gene.
[2] The human T cell of [1] or [1A], which is a naive T cell.
[3] The human T cell of [1], [1A] or [2], wherein the β2 microglobulin (B2M) gene is knocked out.
[4] A method for producing a human T cell capable of sustained proliferation in vitro and/or long-term survival in a recipient after transplantation, comprising performing the following (a) or (b):
   (a) selecting a CD62L-positive CD45RA-positive cell from a human T cell-containing cell population
   (b) applying T cell proliferation stimulation to and culturing the test human T cell-containing cell population, measuring a total ATP production rate and a mitochondrial spare respiratory capacity of the cell population 5 - 6 days after the proliferation stimulation, and selecting a test cell population with a total ATP production rate of 400 pmol/min/10⁵ cells or more and a spare respiratory capacity of 40 pmol/min/10⁵ cells or more.
[5] The method of [4], comprising further expansion culturing human T cells selected by the aforementioned (a) or (b).
[6] The method of [4] or [5], wherein the culturing comprises adding a medium every 2 - 3 days.
[7] A transplantation therapy agent comprising a human T cell having the following characteristics (a) and/or (b):
   (a) CD62L-positive and CD45RA-positive
   (b) a total ATP production rate of a human T cell-containing cell population of 400 pmol/min/10⁵ cells or more and a mitochondrial spare respiratory capacity of 40 pmol/min/10⁵ cells or more, 5-6 days after the application of T cell proliferation stimulation to the cell population.
[8] The agent of [7], wherein the human T cell is a low-immunogenic genetically engineered cell.
[9] The agent of [7] or [8], wherein the human T cell is a CAR-T cell or TCR-T cell.
[10] A method for producing a low-immunogenic human T cell, comprising
   (1) providing a human T cell having the following characteristics (a) and/or (b),
      (a) CD62L-positive and CD45RA-positive
      (b) a total ATP production rate of a human T cell-containing cell population of 400 pmol/min/10⁵ cells or more and a mitochondrial spare respiratory capacity of 40 pmol/min/10⁵ cells or more, 5-6 days after the application of T cell proliferation stimulation to the cell population
   (2) deleting an HLA class I molecule in the human T cell or a progenitor cell thereof, and
   (3) introducing exogeneous ST6GALNAC6 gene into the human T cell or a progenitor cell thereof.
[10A] The method of [10], comprising deleting the TRAC gene in the aforementioned human T cell or a progenitor cell thereof.
[11] The method of [10] or [10A], wherein the human T cell having the aforementioned characteristics (a) and/or (b) is a naive T cell.
[12] The method of [10], [10A] or [11], wherein the HLA class I molecule is deleted by knocking out the B2M gene.
[13] A transplantation therapy agent comprising the human T cell of any of [1], [1A], [2], and [3], or a human T cell obtained by the method of any of [10], [10A], [11], and [12].
[14] The agent of [13], wherein the human T cell is a CAR-T cell or TCR-T cell.

### [Advantageous Effects of Invention]

According to the present invention, a highly versatile human T cell can be provided that is not rejected by T cells or NK cells from the recipient side when allogeneically transplanted, does not attack non-target cells of the recipient, does not cause GVHD, and can be used to produce a safe genetically modified T cell therapeutic agent. According to the present invention, moreover, a human T cell is provided that can be efficiently maintained and expanded for a long time in vitro and can stably survive in the body for a long time after transplantation. Furthermore, by evaluating human T cells by using a mitochondrial activity indicator, a human T cell suitable for transplantation can be selected without the need for an operation that can damage cells, such as FACS.

### [Brief Description of Drawings]

Fig. 1 is a diagram showing the differentiation of memory T cells and the expression of surface antigen markers at each differentiation stage.
Fig. 2 is a diagram showing the culture protocol for the activation and expansion and proliferation of human T cells in Example 1.
Fig. 3 is a diagram showing the results of FACS analysis of human T cells after awakening from cryopreservation after expansion and proliferation in Example 1.
Fig. 4 is a diagram showing that the B2M gene is knocked out in most T cells in the human T cell population in Example 1, by using CRISPR-Cas9 technology.
Fig. 5 is a diagram showing the purification of NK cells (CD3-negative, CD56-positive, CD328 (Siglec-7)-positive).
Fig. 6 is a diagram showing that the human T cell of the present invention obtained in Example 2 is able to avoid attack by NK cells.
Fig. 7 is a diagram showing the culture protocol for the activation and expansion and proliferation of human T cells in Example 3.
Fig. 8 is a diagram showing the results of FACS analysis of human T cells after awakening from cryopreservation after expansion and proliferation in Example 3.
Fig. 9 is a diagram showing that the human T cell population obtained in Example 3 has a higher proportion of naive T cells or stem cell-like memory T cells than the human T cell population obtained in Example 1.
Fig. 10 is a diagram showing that the B2M gene is knocked out in most T cells in the human T cell population in Example 3, by using CRISPR-Cas9 technology.
Fig. 11 is a diagram showing the purification of NK cells (CD3-negative, CD56-positive, CD328 (Siglec-7)-positive).
Fig. 12 is a diagram showing that the human T cell of the present invention obtained in Example 4 is able to avoid attack by NK cells.

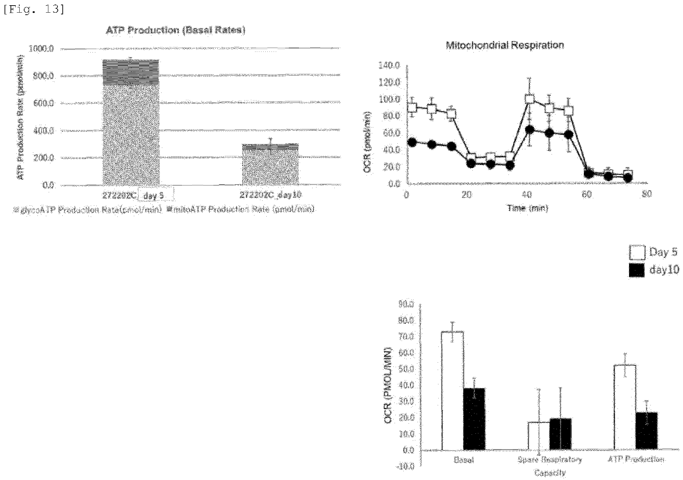

Fig. 13 is a diagram showing the ATP production rate (left panel; upper dark-colored area indicates ATP production rate by mitochondria, and lower light-colored area indicates ATP production rate by glycolysis) of human T cells (Lot 1) on days 5 and 10, and the mitochondrial oxygen consumption rate (OCR) in response to various drugs (right panel; upper graph shows time-dependent changes in OCR when oligomycin, FCCP, and rotenone/antimycin A were added in this order, and lower graph shows, from the left, OCR during basal respiration, spare respiratory capacity, and OCR coupled to ATP production).

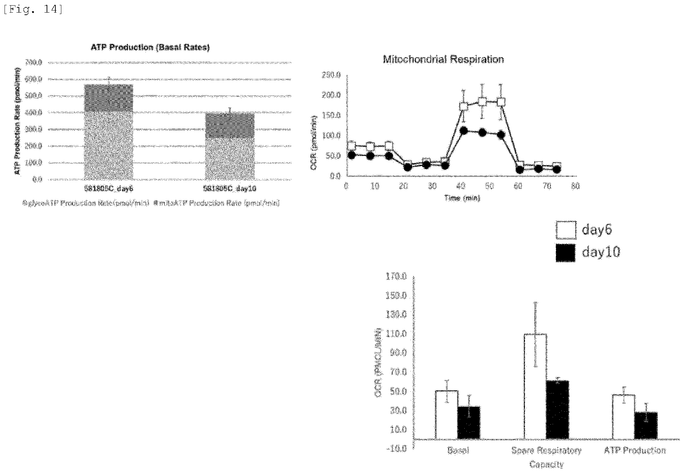

Fig. 14 is a diagram showing the ATP production rate (left panel; upper dark-colored area indicates ATP production rate by mitochondria, and lower light-colored area indicates ATP production rate by glycolysis) of human T cells (Lot 2) on days 6 and 10, and the mitochondrial oxygen consumption rate (OCR) in response to various drugs (right panel; upper graph shows time-dependent changes in OCR when oligomycin, FCCP, and rotenone/antimycin A were added in this order, and lower graph shows, from the left, OCR during basal respiration, spare respiratory capacity, and OCR coupled to ATP production).

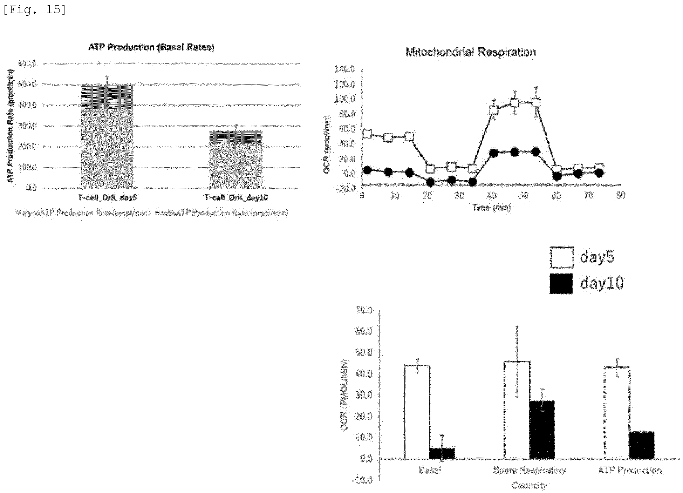

Fig. 15 is a diagram showing the ATP production rate (left panel; upper dark-colored area indicates ATP production rate by mitochondria, and lower light-colored area indicates ATP production rate by glycolysis) of human T cells (Lot 3) on days 5 and 10, and the mitochondrial oxygen consumption rate (OCR) in response to various drugs (right panel; upper graph shows time-dependent changes in OCR when oligomycin, FCCP, and rotenone/antimycin A were added in this order, and lower graph shows, from the left, OCR during basal respiration, spare respiratory capacity, and OCR coupled to ATP production).

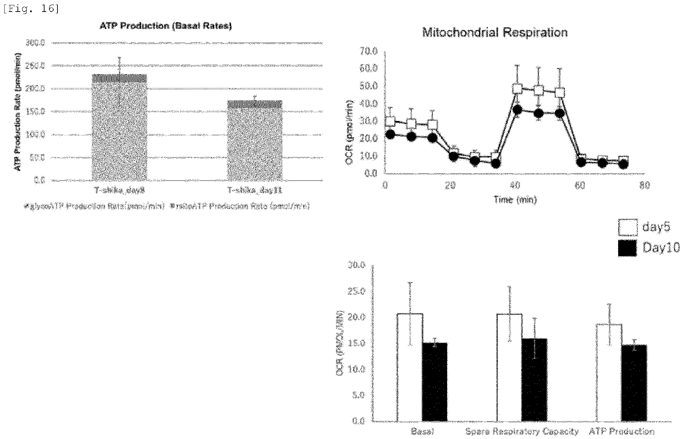

Fig. 16 is a diagram showing the ATP production rate (left panel; upper dark-colored area indicates ATP production rate by mitochondria, and lower light-colored area indicates ATP production rate by glycolysis) of human T cells (Lot 4) on days 5 and 10, and the mitochondrial oxygen consumption rate (OCR) in response to various drugs (right panel; upper graph shows time-dependent changes in OCR when oligomycin, FCCP, and rotenone/antimycin A were added in this order, and lower graph shows, from the left, OCR during basal respiration, spare respiratory capacity, and OCR coupled to ATP production).
Fig. 17 is a diagram showing the proliferation curves of human T cells (Lot 1 to Lot 4).
Fig. 18 is a diagram showing the results of FACS analysis of human T cells (Lot 2 to Lot 5) on day 7.
Fig. 19 is a diagram showing the daily change in the proportion of undifferentiated cells in human T cells (Lot 3) in vitro (upper panel) and the retention of cells in the body after transplantation into humanized mice (lower panel). Mouse 1 died within 4 weeks after transplantation.
Fig. 20 is a diagram showing the retention of human T cells (Lot 4) in the body after transplantation into humanized mice (lower panel). CD3-positive CD62L-positive cells disappeared within 4 weeks after transplantation in all mice except mouse 2.
Fig**.** 21 is a diagram showing the effect of medium addition intervals on the proliferation of human T cells.
Fig**.** 22 is a diagram showing that the B2M gene and TRAC gene are knocked out in most T cells in the human T cell population in Example 7, by using CRISPR-Cas9 technology.
Fig**.** 23 is a diagram showing that the human T cell of the present invention obtained in Example 7 is able to avoid attack by NK cells.

### [Description of Embodiments]

The present invention provides a CD62L-positive CD45RA-positive human T cell that lacks HLA class I molecules and contains an exogeneous ST6GALNAC6 gene, or a human T cell equivalent thereto (hereinafter also to be referred to as "the human T cell of the present invention"). As used herein, being "equivalent to CD62L-positive CD45RA-positive human T cell" means that it has long-term maintenance ability in vitro and/or long-term retention ability in the body after transplantation that are equivalent to those of CD62L-positive CD45RA-positive human T cells, and is defined as being above the thresholds for the ATP production rate and mitochondrial spare respiratory capacity described later. The "long-term maintenance ability" and "long-term retention ability in the body" are not particularly limited as long as the maintenance of an undifferentiated state and retention in the body are maintained for a significantly long period of time compared with CD62L-negative and/or CD45RA-negative human T cells, or human T cells below the aforementioned threshold values (control cells). For example, the long-term maintenance ability in vitro is characterized by the retention of CD62L-positive cells for 2 weeks or more, preferably 4 weeks or more, and more preferably 6 weeks or more after the application of proliferation stimulation, or by the CD62L positivity rate of 90% or more 1 week after the application of proliferation stimulation. The long-term retention in the body is characterized, for example, by the retention of human T cells in the blood of an immunodeficient mouse for not less than 4 weeks, preferably not less than 6 weeks, after transplantation.

When allogeneically transplanted into the body of a recipient, the human T cell of the present invention is not subject to the immune rejection reaction that generally occurs when the recipient recognizes the cells as non-self, i.e., attack by T cells or NK cells of the recipient, or even if an immune rejection reaction occurs, the reactivity is extremely low. Furthermore, since the human T cell of the present invention does not recognize the cells of the recipient as non-self and does not attack them, it does not cause GVHD. Therefore, the human T cell of the present invention can be a versatile donor cell for allogeneic transplantation.

The human T cell of the present invention can be produced by deleting HLA class I molecules in CD62L-positive CD45RA-positive human T cells or human T cells equivalent thereto, or any human cells that can be differentiated into said cells, and introducing an exogenous ST6GALNAC6 gene (and, where necessary, further differentiating into CD62L-positive CD45RA-positive or equivalent human T cells). In the present invention, the "T cell" refers to a type of leukocyte found in lymphoid organs or peripheral blood, and the like, and is one classification of lymphocytes characterized by differentiating and maturing mainly in the thymus and expressing TCR. Examples of the T cell that can be used in the present invention include cytotoxic T cells (CTLs) that are CD8 positive cells, helper T cells that are CD4 positive cells, and the like, and cytotoxic T cells are preferred.

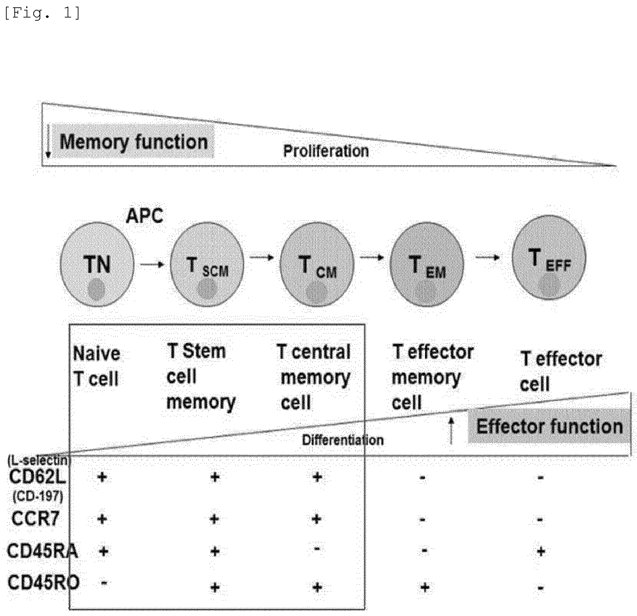

In adoptive immunotherapy such as CAR-T or TCR-T cell therapy, whether cancer cells can be eradicated is closely related to the long-term viability of transplanted T cells. Memory T cells are inherently capable of surviving for a long period of time by repeating self-replication, but their life span is finite, and they gradually lose their viability as the cells proliferate (Fig. 1). Changes are observed in the marker profile expressed on the cell surface during the differentiation process, and the most undifferentiated naive T cells and stem cell-like memory T cells express surface antigens such as CD62L, CD45RA, CCR7, CD27, CD28, and CD127. Therefore, CD62L-positive CD45RA-positive human T cell fractions contain a large amount of the most undifferentiated memory fraction, and long-term survival and therapeutic effects after transplantation can be expected.

CD62L-positive CD45RA-positive human T cells can be isolated, for example, from collected human peripheral blood or mononuclear cells (PBMCs) isolated therefrom, by using a conventional method such as flow cytometry, with the expression of two or more of the surface antigens expressed in the above-mentioned naive T cells or stem cell-like memory T cells, or the non-expression of CD45RO expressed in central memory T cells or effector memory T cells as an indicator.

T cells can be activated and expanded from a human T cell-containing sample such as human PBMC by conventional methods. For example, a human T cell-containing sample can be cultured by adding to the medium a molecule that interacts with a surface molecule of T cell to promote T cell activation and/or proliferation. Examples of such molecule include CD3, which conjugates with TCR and is responsible for TCR-mediated signal transduction, and a molecule that specifically binds to surface molecules known as costimulatory factors for T cell activation, such as CD28, ICOS, CD137, OX40, CD27, GITR, BAFFR, TACI, BMCA, and CD40L, and transmits activation/proliferation signals and co-signals into T cells. Preferred examples include antibodies against CD3 and/or antibodies against CD28. These molecules may be used alone or in the form of a complex **(e.g.,** TransAct (Milteny Biotech), Dynabeads Human T-Activator CD3/CD28 (ThermoFisher Scientific)) bound to a carrier (e.g., Dynabeads (registered trademark) (ThermoFisher Scientific)).

When applying proliferation stimulation to the T cells by using anti-CD3 antibody and/or anti-CD28 antibody, cytokines such as IL-2, IL-7, and IL-15 may be further added to the medium.

As the medium to which the proliferation stimulating factor is added, various known cell culture media can be appropriately selected and used. A specific example of a medium suitable for culturing T cells is RPMI1640 medium. In addition to the proliferation stimulating factor, the medium may be supplemented with various serums (e.g., human serum and fetal bovine serum), amino acids (e.g., L-glutamine), antibiotics (e.g., penicillin and streptomycin), other cytokines, and the like. Alternatively, commercially available T cell activation media (e.g., KBM501 (Kohjin Bio)) and expansion media (e.g., KBM502 (Kohjin Bio)) can also be used.

While the seeding density of T cells when applying proliferation stimulation is not particularly limited, it is preferable to perform high-density culture in order to increase the contact opportunities of T cells, promote clustering, and apply efficient proliferation stimulation.

Since activation-induced cell death may be induced when proliferation stimulation continues for a long time. In order to avoid this, the proliferation stimulating factor may be removed or reduced from the culture system after a certain period of time has elapsed since the start of the proliferation stimulation. After culturing for a period of time sufficient to promote T cell proliferation, the cell density may be reduced to prevent continued proliferation stimulation due to cell-cell interactions. The cell density may be reduced by recovering the cells and reseeding them at a low density. More simply, it can be performed by adding the medium. The frequency of medium addition is not particularly limited, but from the aspect of proliferation efficiency, the medium is preferably added every 2 to 3 days.

The pH of the medium is preferably about 6 to about 8, and the CO₂ concentration is preferably about 2 to 5%. The culture temperature is generally about 30 to 40°C, preferably about 37°C. Aeration and stirring may be performed as necessary.

The present inventors have evaluated the total ATP production rate and mitochondrial spare respiratory capacity of a human T cell-containing cell population, such as PBMC, 5 to 6 days after the application of proliferation stimulation to the cell population, and found that, when the total ATP production rate is 400 pmol/min/10⁵ cells or more and the mitochondrial spare respiratory capacity is 40 pmol/min/10⁵ cells or more, long-term in vitro maintenance proliferation and long-term in vivo retention after transplantation can be expected, whereas when one or both of them is/are below the threshold values, sufficient in vitro maintenance proliferation and long-term in vivo retention after transplantation cannot be expected. As used herein, "long-term maintenance proliferation" and "long-term in vivo retention" have the same meanings as those described above. The present inventors have also found that 90% or more of human T cells with both indicators above the threshold values are CD62L-positive undifferentiated cells, whereas human T cells with at least one indicator below the threshold value have a markedly reduced CD62L positivity rate. These findings indicate that, by using the total ATP production rate and the spare mitochondrial respiratory capacity as indicators, an undifferentiated T cell fraction suitable for transplantation can be selected from a human T cell-containing cell population, without performing a cell sorting operation such as FACS.

Therefore, the present invention also provides a production method of a human T cell capable of sustained proliferation in vitro and/or long-term survival in a recipient after transplantation. The method characteristically includes performing the following (a) or (b):
(a) selecting a CD62L-positive CD45RA-positive cell from a human T cell-containing cell population
(b) applying T cell proliferation stimulation to and culturing the test human T cell-containing cell population, measuring a total ATP production rate and a mitochondrial spare respiratory capacity of the cell population 5 - 6 days after the proliferation stimulation, and selecting a test cell population with a total ATP production rate of 400 pmol/min/10⁵ cells or more and a spare respiratory capacity of 40 pmol/min/10⁵ cells or more.

In the aforementioned (b), it is more preferable to select test cells in which the ratio of the mitochondrial ATP production rate to the total ATP production rate is 1/3 or less.

The total ATP production rate, the mitochondrial spare respiratory capacity, and the mitochondrial ATP production rate may be evaluated by any method known per se**.** For example, the evaluation can be performed by measuring the oxygen consumption rate (OCR) and extracellular acidification rate (ECAR; rate of mPH change in the well) using an extracellular flux analyzer from Agilent Technologies (e.g.,
https://www.primetech.co.jp/products/tabid/90/pdid/218/language /ja-JP/Default.aspx#ProductAttachDetail7;
https://www.agilent.com/cs/library/whitepaper/public/whitepaper -quantify-atp-production-rate-cell-analysis-5991-9303en-agilent.pdf;
https://www.agilent.com/cs/library/usermanuals/public/103591-400_Seahorse_XFp_ATP_Rate_Assay_Kit_User_Guide.pdf and the like). In other words, since the reaction in glycolysis that converts one molecule of glucose into two molecules of lactic acid produces two molecules of ATP and protons, the ATP production rate by glycolysis (glycoATP) is equal to the proton efflux rate by glycolysis (glycoPER). glycoPER is the total proton efflux rate (PER) minus the mitochondrial proton efflux rate (mitoPER). PER is calculated using the following formula. PER (pmol/min) = ECAR (mpH/min) x BF (mmol/L/pH) x chamber volume (µL) x Kvol
wherein BF (Buffer Factor) is a measure of buffer capacity that takes into account the buffer capacity of the medium and the sensor system, and the BF value is set in advance for standard assay medium. In the formula, Kvol is an empirically obtained coefficient used to calculate PER in the measurement chamber, and is set in advance depending on the type of device (XF, XFp, XFe24, XFe96) (e.g., 1.6 for XF).
   mitoPER is calculated using the following formula. mitoPER (pmol H+/min) = mitoOCR (pmol O2/min) × CCF (pmol H+/pmol O2)
wherein mitoOCR is the oxygen consumption rate by mitochondria, and CCF indicates the CO₂ contribution coefficient, which is set in advance depending on the type of device (e.g., 0.61 for XF).
   mitoOCR is the value obtained by subtracting the OCR when the electron transport system is completely inhibited with rotenone/antimycin A from the OCR during basal respiration. The OCR coupled with ATP production in oxidative phosphorylation is the value obtained by subtracting the OCR when complex I is inhibited with oligomycin from the mitoOCR.

The ATP production rate by mitochondria (mitoATP) is calculated using the following formula. mitoATP (pmol ATP/min) = mitoOCR (pmol O2/min) × 2 × P/O (pmol ATP/pmol O) wherein P/O is the amount of ATP molecules produced per oxygen atom, and is calculated for each nutrient based on the stoichiometry of each nutrient metabolized and experimental results. A reliable evaluation is possible by using a P/O ratio of 2.75.

The total ATP production rate of the cells is calculated as the sum of glycoATP and mitoATP obtained as described above.

The mitochondrial spare respiratory capacity is calculated as the value obtained by subtracting the OCR during basal respiration from the OCR when the proton gradient is eliminated by a deconjugating agent such as FCCP and oxygen consumption by complex IV is maximized.

In the present invention, a total ATP production rate of 400 pmol/min/10⁵ cells or more and a spare respiratory capacity of 40 pmol/min/10⁵ cells or more means that the values measured and calculated using an extracellular flux analyzer XF HS Mini from Agilent Technologies are equal to or greater than the threshold value.

In one embodiment, the activation culture of T cells can be performed for, for example, 4 to 7 days, preferably 5 to 6 days. Thereafter, CD62L-positive CD45RA-positive human T cells or human T cells equivalent thereto can be efficiently obtained by conducting expansion culture for, for example, 5 days or more, preferably 7 days or more. The obtained cells can be frozen and stored by a conventional method until the genetic manipulation described below.

In another embodiment, the activation culture of T cells can be performed for, for example, 3 to 4 days. Thereafter, expansion culture for, for example, 1 to 4 days, preferably 2 to 3 days, is performed, and the cells can be cryopreserved by a conventional method. After thawing the frozen cells, for example, using a mixed medium of an activation medium and an expansion medium, awakening and expansion culture of the cells were performed simultaneously, and then genetic manipulation as described below is performed, followed by expansion culture, whereby CD62L-positive CD45RA-positive human T cells or human T cells equivalent thereto can be efficiently obtained. The awakening and expansion culture can be performed for, for example, 3 to 5 days, and the subsequent expansion culture can be performed for, for example, 2 days or more, preferably 4 days or more.

The CD62L-positive CD45RA-positive human T cells or human cells that can be induced to differentiate into equivalent human T cells may be lymphocyte progenitor cells, including pluripotent cells. Examples of lymphocyte progenitor cells, including pluripotent cells, include embryonic stem cells (ES cells), induced pluripotent stem cells (iPS cells), embryonic tumor cells (EC cells), embryonic germ stem cells (EG cells), hematopoietic stem cells, multipotent progenitor cells that have lost the ability to self-replicate (MPP), myelo-lymphoid common progenitor cells (MLP), myeloid progenitor cells (MP), granulocyte mononuclear progenitor cells (GMP), macrophage-dendritic cell progenitor cells (MDP), and dendritic cell progenitor cells (DCP). Pluripotent stem cells, such as ES cells and iPS cells, are preferred.

The method of inducing differentiation of the aforementioned progenitor cells into T cells can be appropriately selected from various known differentiation induction methods. For example, known methods of inducing differentiation of human pluripotent stem cells into T cells include the method of inducing T cells from human ES cells described in Timmermans, F. et al., J. Immunol., 2009, 182, 68879-6888. In addition, human iPS cells established from human peripheral blood T lymphocytes can be induced to differentiate into T cells by the method described in Nishimura, T. et al., ]Cell Stem Cell, 2013, 12, 114-126.

The human T cells of the present invention lack HLA class I (HLA-A, B, C, E, G, F) molecules. HLA class I molecules are transmembrane proteins that all nucleated cells possess. In particular, HLA class Ia (HLA-A, B, C) has not less than several thousand various polymorphisms, and therefore, the HLA class Ia expressed on the cell surface by each individual is highly diverse. This diversity plays the most important role in distinguishing self from non-self. HLA class I molecules presented on the cell surface are recognized by T cell receptors (TCR) on the surface of CTL. Cells presenting the recognized HLA class I are recognized as exogeneous bodies by CTLs and eliminated by the immune system. Therefore, cells with insufficient expression of HLA class I are suitable as a source of transplant cells since they can avoid recognition by CTL.

HLA class I molecule is a heterodimer consisting of an α chain encoded by each gene of HLA class I and a β chain, which is β2 microglobulin (B2M). Therefore, to eliminate MHC class I molecules, each of the HLA class I genes encoding the α chains may be knocked out, or the B2M gene encoding the β chain common to all HLA class I molecules may be knocked out. In the former case, six types of HLA class I genes must be knocked out. In order to eliminate all HLA class I molecules, it is simpler to knock out the B2M gene.

In the present specification, to delete (knock out) an endogenous gene means to destroy or remove the endogenous gene so that it is unable to produce complete mRNA.

As a specific means for deleting an endogenous gene, a method including isolating genomic DNA from a target cell, in which an endogenous gene is to be deleted, by a conventional method, then, for example, (1) disrupting the function of the exon or promoter by inserting another DNA fragment (e.g., a drug resistance gene or a reporter gene) into the exon portion or promoter region of the endogenous gene, (2) deleting the endogenous gene by excising all or part of the gene using the Cre-loxP system or the Flp-frt system, (3) inserting a stop codon into the protein coding region to disable complete protein translation, or (4) inserting a DNA sequence that terminates gene transcription (e.g., a polyA addition signal, etc.) into the transcription region to disable complete mRNA synthesis, thereby constructing a DNA strand having a DNA sequence that inactivates the gene (hereinafter to be abbreviated as a targeting vector for gene deletion), and incorporating the vector into the endogenous gene locus of the target cell by homologous recombination may be preferably used.

The homologous recombinant cell can be obtained, for example, by introducing the above-mentioned targeting vector into the target cell.

For example, when the targeting vector for gene deletion is designed to destroy the function of the exon or promoter of the endogenous gene by inserting another DNA fragment into the exon part or promoter region, the vector can have the following configuration.

First, in order to insert other DNA fragment into the exon or promoter part of endogenous gene by homologous recombination, the targeting vector for gene deletion must contain sequences (5' arm and 3' arm) that are respectively homologous to the target site 5' upstream and 3' downstream of the DNA fragment of said other gene.

There are no particular limitations on other DNA fragment to be inserted. When a drug resistance gene or reporter gene is used, target cells in which the targeting vector for gene deletion has been incorporated into the chromosome can be selected using drug resistance or reporter activity as an indicator. Examples of the drug resistance gene include, but are not limited to, neomycin phosphotransferase II (nptII) gene and hygromycin phosphotransferase (hpt) gene, and examples of the reporter gene include, but are not limited to, β-galactosidase (lacZ) gene and chloramphenicol acetyltransferase (cat) gene.

It is preferable that the drug resistance or reporter gene is under the control of any promoter that can function in the target cell. Examples include viral promoters such as the SV40-derived early promoter, cytomegalovirus (CMV) long terminal repeat (LTR), Rous sarcoma virus (RSV) LTR, murine leukemia virus (MoMuLV) LTR, and adenovirus (AdV)-derived early promoter, as well as β-actin gene promoter, PGK gene promoter, and transferrin gene promoter. However, when a drug resistance or reporter gene is inserted into an endogenous gene so as to be under the control of the endogenous promoter of the HLA class I gene, the targeting vector for gene deletion does not need a promoter for controlling the transcription of the gene.

Furthermore, it is preferable that the targeting vector for gene deletion has a sequence (polyadenylation (polyA) signal, also called a terminator) that terminates the transcription of mRNA from the gene, at the downstream of the drug resistance or reporter gene. For example, a terminator sequence derived from a virus gene or from various mammalian or avian genes can be used. Preferably, an SV40-derived terminator is used.

Generally, most gene recombinations in cells are non-homologous, and the introduced DNA is randomly inserted at any position in the chromosome. Therefore, it is not possible to efficiently select only clones in which homologous recombination has occurred at the target site by selection such as detecting the expression of drug resistance or reporter genes (positive selection), and it is necessary to confirm the integration site for all selected clones by Southern hybridization or PCR. Therefore, if, for example, the herpes simplex virus-derived thymidine kinase (HSV-tk) gene, which confers ganciclovir sensitivity, is linked to the outside of the sequence homologous to the target site of the gene deletion targeting vector, cells into which the vector is randomly inserted will have the HSV-tk gene and will be unable to grow in a ganciclovir-containing medium, but cells into which the vector is targeted to an endogenous locus by homologous recombination will not have the HSV-tk gene and will therefore be resistant to ganciclovir and will be selected (negative selection). Alternatively, if, for example, the diphtheria toxin gene is linked instead of the HSV-tk gene, cells into which the vector is randomly inserted will die due to the toxin they produce, and homologous recombinants can also be selected in the absence of drugs.

The introduction of a targeting vector for gene deletion into a target cell can be performed using any of calcium phosphate coprecipitation method, electroporation method, lipofection method, retrovirus infection method, aggregation method, microinjection method, gene gun (particle gun) method, and DEAE-dextran method. However, as mentioned above, most gene recombinations in cells are non-homologous, and homologous recombinants are obtained only rarely. Therefore, electroporation method is generally selected because it can easily treat a large number of cells. For electroporation, the same conditions as those used for gene introduction into normal animal cells can be used. For example, target cells in the logarithmic growth phase can be treated with trypsin to disperse them into single cells, then suspended in a medium to a density of 10⁶ - 10⁸ cells/ml and transferred to a cuvette, followed by the addition of 10-100 µg of the targeting vector for gene deletion and the application of an electric pulse of 200-600 V/cm.

Target cells into which a targeting vector for gene deletion has been incorporated can also be tested by screening, using Southern hybridization or PCR, chromosomal DNA isolated and extracted from colonies obtained by culturing single cells. When a drug resistance gene or reporter gene is used as another DNA fragment, transformants can be selected at the cell stage by using the expression of the gene as an indicator. For example, when a vector containing the nptII gene is used as a positive selection marker gene, the target cells after gene introduction are cultured in a medium containing a neomycin antibiotic such as G418, and the resistant colonies that emerge are selected as candidates for transformants. When a vector containing the HSV-tk gene is used as a negative selection marker gene, the cells are cultured in a medium containing ganciclovir, and the resistant colonies that emerge are selected as candidates for homologous recombinant cells. The obtained colonies are transferred to culture plates and repeatedly treated with trypsin and the medium is repeatedly exchanged, after which some are left for culture and the remaining cells are subjected to PCR or Southern hybridization to confirm the presence of the introduced DNA.

In addition, when a virus is used as a targeting vector for gene deletion, a method can be used in which a positive selection marker gene is inserted between the 5' and 3' arms and a target cell is infected with a virus containing DNA containing a negative selection marker gene on the outside of the arms. For example, when a retrovirus or lentivirus is used, cells are seeded in an appropriate culture vessel such as a dish and the like, a viral vector is added to the culture medium (polybrene may be coexisted when desired), and after culturing for 1 to 2 days, a selection agent is added as described above, and the culture is continued to select cells into which the vector has been incorporated.

Another preferred embodiment for deleting an endogenous gene is the CRISPR-Cas9 (Clustered Regularly Interspaced Short Palindromic Repeats CRISPR-Associated proteins 9) system. According to the CRISPR-Cas9 system, a gene mutation can be introduced by cutting any region on the genome DNA by using Cas9, which is a genomic DNA cleavage enzyme, and sgRNA, which is an RNA molecule that recognizes a targeted site on the genome. In/del bases that occur during the repair process in vivo accompanying genomic DNA cleavage cause a frameshift in DNA encoding amino acids, resulting in deletion of the target endogenous gene.

Cas9 functions as an endonuclease that recognizes the protospacer adjacent motif (PAM) sequence in DNA and cleaves it at the upstream thereof. Since Cas9 contains two functional domains with endonuclease activity, it can cleave double-stranded DNA to produce blunt ends. For example, specifically, Cas9 forms a complex with a single-stranded nucleic acid (sgRNA) containing a base sequence (CRISPR-RNA (crRNA)) that specifically binds to an endogenous gene, and generates a double-stranded break (DSB) on the 5' side of the PAM in the endogenous gene. Therefore, in the present invention, Cas9 refers to a protein that forms a complex with a guide RNA and has double-stranded DNA cleavage activity.

Examples of Cas9 include, but are not limited to, SpCas9 derived from Streptococcus pyogenes, StCas9 derived from Streptococcus thermophilus, and NmCas9 derived from Neisseria meningitidis.

Cas9 may also be a mutant Cas9. The mutant Cas9 is not particularly limited as long as it is a protein maintaining the ability to form a complex with a guide RNA and having a mutation in which one of the two functional domains having endonuclease activity contained in Cas9 is inactivated. Examples of such mutant Cas9 include Cas9 having one mutation selected from the group consisting of a mutation in which the 10th aspartic acid of Cas9 is replaced with alanine (D10A mutation), a mutation in which the 840th histidine is replaced with alanine (H840A mutation), and/or a mutation in which the 863rd asparagine is replaced with alanine (N863A mutation).

The crRNA is not particularly limited as long as it has a base sequence complementary to the endogenous gene and is adjacent to the 5' side of the PAM in the endogenous gene. The length of the crRNA base sequence is not particularly limited as long as it can ensure specificity for the endogenous gene, and is generally 10 to 30 bases long, preferably 15 to 25 bases long, more preferably 20 bases long. A 20-base-long crRNA that can knock out the human B2M gene is commercially available (e.g., TrueGuide^{™}Synthetic sgRNA Assay IDs: CRISPR983707_SGM, CRISPR42443_SGM, CRISPR983713_SGM, CRISPR983710_SGM (Thermio Fisher Scientific)), and these can also be used.

PAM varies depending on the type of Cas9. Examples include NGG (N is A, G, T, or C, hereinafter the same) when Cas9 derived from Streptococcus pyogenes (SpCas9) is used, NNAGAAW when Cas9 derived from Streptococcus thermophilus (StCas9) is used, and NNNNGATT when Cas9 derived from Neisseria meningitidis (NmCas9) is used.

Single-stranded nucleic acid (sgRNA) containing a base sequence that specifically binds to an endogenous gene may further contain a base sequence (trans-activating RNA (tracrRNA)) necessary for recruiting Cas9. A known base sequence can be used as the base sequence of tracrRNA. The tracrRNA may be linked directly to the 3' end of the crRNA, or a spacer sequence may be sandwiched between them.

Alternatively, the crRNA and the tracrRNA may be associated via complementary binding (i.e., may be double-stranded nucleic acid). Even when gene editing is performed using such double-stranded nucleic acid, the method of introduction into the target cell described below is the same as that of the introduction of single-stranded nucleic acid.

The introduction of Cas9 and sgRNA into the target cell can be performed according to known means. For example, Cas9 and sgRNA can be introduced into the target cell by inserting a nucleic acid sequence encoding Cas9 and a nucleic acid sequence transcribing sgRNA into an appropriate expression vector, and then introducing the expression vector into the target cell.

Examples of the nucleic acid sequence encoding Cas9 include genomic DNA and synthetic DNA. Genomic DNA encoding Cas9 can be directly amplified by polymerase chain reaction (hereinafter abbreviated as "PCR method") using a primer set complementary to the cas9 gene, using a genomic DNA fraction prepared from the microorganism as a template. The nucleic acid sequence transcribing sgRNA can be prepared by DNA synthesis and PCR.

An expression vector containing a nucleic acid sequence encoding Cas9 and a nucleic acid sequence transcribing sgRNA can be produced, for example, by linking a nucleic acid sequence fragment encoding Cas9 and a nucleic acid sequence fragment transcribing sgRNA at the downstream of a promoter in an appropriate expression vector.

As expression vectors, animal cell expression plasmids (e.g., pA1-11, pXT1, pRc/CMV, pRc/RSV, pcDNAI/Neo); animal virus vectors such as retrovirus, lentivirus, adenovirus, and adeno-associated virus are used.

As promoters, any promoter appropriate for the host used to express the gene can be used.

For example, SRα promoter, SV40 promoter, LTR promoter, CMV (cytomegalovirus) promoter, RSV (Rous sarcoma virus) promoter, MoMuLV (Moloney murine leukemia virus) LTR, HSV-TK (herpes simplex virus thymidine kinase) promoter, and the like are used. Among these, the CMV promoter, SRα promoter, and the like are preferred.

In addition to the above, the expression vector may contain an enhancer, a polyA addition signal, a selection marker, an SV40 origin of replication (hereinafter sometimes abbreviated as SV40 ori), and the like when desired. Examples of the selection marker include dihydrofolate reductase gene (hereinafter sometimes abbreviated as dhfr, methotrexate (MTX) resistance), neomycin resistance gene (hereinafter sometimes abbreviated as neor, G418 resistance), and the like.

By introducing the expression vector containing the above-mentioned nucleic acid sequence encoding Cas9 and the nucleic acid sequence transcribing sgRNA into the target cell and culturing same, Cas9 and sgRNA form a complex in the target cell, and the endogenous gene targeted by the sgRNA is cleaved. In the endogenous gene cleaved by Cas9, small insertions and/or deletions (in/dels) are introduced into the endogenous gene during DSB repair by the non-homologous end joining (NHEJ) pathway, causing a frameshift and site-specific mutation or destruction of the endogenous gene.

In addition, the Cas9 protein itself may be used as Cas9 without using the expression vectors exemplified above. The endogenous gene targeted by the sgRNA can also be cleaved by combining the Cas9 protein with an sgRNA to form a complex and introducing same into a target cell.

The method using the CRISPR-Cas9 system has been described in detail as a genome editing method for deleting an endogenous gene. A person skilled in the art can similarly delete a desired endogenous gene by using other genome editing techniques such as other known CRISPR techniques, ZFN, and TALEN.

HLA class II is a transmembrane protein found only in antigen-presenting cells such as macrophages, dendritic cells, and B cells. HLA class II presents peptide antigens derived from extracellular proteins, including proteins of extracellular pathogens taken up by immune cells by phagocytosis, on the cell surface. The peptide antigens presented by HLA class II interact with the TCR of CD4-positive helper T cells to activate the CD4-positive helper T cells. The activated T cells recognize and activate B cells that similarly present peptide antigens by HLA class II, thereby inducing events such as recruitment of phagocytes, local inflammation, humoral responses, and activation of CTL. Therefore, cells with insufficient expression of HLA class II are suitable as a normal transplant cell source because they can avoid the occurrence of the above events. However, since the human T cells of the present invention do not basically express HLA class II, it is not essential to delete HLA class II.

When HLA class II is also to be deleted, the endogenous genes encoding the α-chain and/or β-chain of HLA class II may be deleted in the same manner as described above, or an endogenous gene encoding a common transcription factor that controls the expression of each gene of HLA class II, such as an endogenous gene encoding RFXANK, an endogenous gene encoding RFX5, an endogenous gene encoding RFXAP, or an endogenous gene encoding CIITA, may be deleted.

The human T cells of the present invention may be deficient in endogenous T cell receptor (TCR) in addition to being deficient in HLA class I molecules. TCR deletion can be achieved by knocking out the TCR α-chain or β-chain constant region (TRAC or TRBC) gene. By deleting the endogenous TCR, it is possible to further suppress GVHD, in which the human cells attack the patient's cells and tissues when the human T cells of the present invention are allografted.

As mentioned above, the human T cells of the present invention are deficient in all HLA class I molecules, which highly suppresses GVHD after transplantation of the T cells and rejection by CTL in the recipient. However, NK cells, which control natural immunity, express many inhibitory receptors that bind to each HLA class I molecule and suppress the activation of NK cells, and cells that do not express any HLA class I molecules are rejected by activated NK cells. Thus, they are inconvenient for use as a cell source for transplantation. For this reason, in conventional low-immunogenic human cells, a method is used in which only the endogenous gene encoding the α chain of HLA class Ia is deleted, or the B2M gene is deleted to delete all HLA class I, and an exogenous gene encoding the α chain of HLA class Ib, such as HLA-E, is introduced to avoid attack by NK cells.

The human T cells of the present invention are designed to avoid attacks from NK cells of the recipient by introducing and forcibly expressing an exogenous ST6GALNAC6 gene. The ST6GALNAC6 gene encodes an enzyme that synthesizes ganglioside DSGb5, which binds to Siglec-7, which is one of the inhibitory receptors on NK cells, and inhibits NK cell activation. Therefore, the human T cells of the present invention that overexpress the ST6GALNAC6 gene can avoid attacks from NK cells of the recipient.

In the present specification, introducing an exogenous gene means making expression of the exogenous gene possible in the target cell by introducing the exogenous gene into a target site in the genome. As a specific means for introducing an exogenous gene, a method including isolating the DNA of the exogenous gene in a conventional manner, inserting a DNA fragment of the exogenous gene into, for example, a target site in the target cell, thereby constructing a DNA strand having a DNA sequence such that the exogenous gene is expressed in the target cell (hereinafter to be abbreviated as a targeting vector for gene introduction), and incorporating the vector into the target site in the target cell by homologous recombination may be preferably used. Since the position of gene insertion is fixed in the homologous recombination method, it is expected that there will be little difference in expression levels between clones and little effect on other genes in the absence of random integration.

The homologous recombinant cell can be obtained, for example, by introducing the above-mentioned targeting vector into the target cell.

For example, when the targeting vector for gene introduction is designed to express an exogenous gene in a target cell by inserting a DNA fragment of the exogenous gene (which can be cloned by a conventional method based on the sequence information of the cDNA of the human ST6GALNAC6 gene (see, for example, Reefseq NM_013443)) into a target site, the vector can have the following configuration.

First, in order to insert the DNA fragment of the exogenous gene into the target site by homologous recombination, the targeting vector for gene introduction must contain sequences (5' arm and 3' arm) that are respectively homologous to the target site 5' upstream and 3' downstream of the DNA fragment of the exogenous gene.

In order to select a target cell in which the targeting vector for gene introduction has been incorporated into the chromosome, it is preferable that the targeting vector for gene introduction contains a drug resistance gene or a reporter gene in addition to the exogenous gene to be inserted. Here, the drug resistance gene and the reporter gene may be the same as those used in the targeting vector for gene deletion.

It is preferable that the drug resistance or reporter gene is under the control of any promoter that can function in the target cell. Here, the promoter may be the same as that used in the targeting vector for gene deletion.

The targeting vector for gene introduction preferably has a polyA signal downstream of the drug resistance or reporter gene, and may be the same as that used in the targeting vector for gene deletion.

It is also preferable to link the HSV-tk gene or the diphtheria toxin gene to the outside of the sequence homologous to the target site of the targeting vector for gene introduction, since this allows the selection of cells targeted to the target site by homologous recombination.

The targeting vector for gene introduction may be introduced into the target cell by the same method as that used for the targeting vector for gene deletion.

Homologous recombinant cells incorporating a targeting vector for gene introduction may be selected by the same method as the method for selecting homologous recombinant cells incorporating a targeting vector for gene deletion.

In addition, when a virus is used as a targeting vector for gene introduction, a method can be used in which an exogeneous gene and a positive selection marker gene are inserted between the 5' and 3' arms and a target cell is infected with a virus containing DNA containing a negative selection marker gene on the outside of the arms. The virus, the method of infecting the cells, and the method of selecting the cells in which the vector has been incorporated may be the same as the virus and method used for the targeting vector for gene deletion.

The target site of the gene introduction targeting vector is not particularly limited as long as it can make the exogenous gene expressible in the target cell, and an example of such site is a safe harbor region in the genome. Here, the safe harbor region is a site in which the phenotype does not change even if an exogenous gene is incorporated, and is selected as a target site for incorporating an exogenous gene into cells to be used as a pharmaceutical product. Examples of such safe harbor region include AAVS1 (Adeno-associated virus integration site 1) region, CCR5 (C-C chemokine receptor 5) region, and ROSA26 region. Introduction of an exogenous gene into a site other than the safe harbor region is not necessarily preferable because the gene at the introduced site may be destroyed, resulting in an unexpected phenotype, or the expression of the introduced exogenous gene may be suppressed. When an exogenous gene is introduced into a safe harbor region, the integration site of the exogenous gene is fixed. Thus, it is expected that there will be less difference in the expression level of the exogenous gene between the obtained homologous recombinants and less influence on other genes.

Another embodiment for introducing an exogenous gene is the PiggyBac method. In the PiggyBac method, a transposon vector into which a DNA fragment containing an exogenous gene has been integrated and a transposase expression vector that expresses a transposase are used. The genes and the like contained in the transposon vector and the transposase expression vector may be present separately in the above-mentioned separate vectors, or may be contained in a single vector. The transposon vector and transposase expression vector can have, for example, the following configuration.

In order to excise a DNA fragment containing an exogenous gene from the transposon vector by transposase, the transposon vector contains terminal inverted repeats at the 5' upstream and 3' downstream of the DNA fragment containing the exogenous gene. The transposase recognizes the terminal inverted repeats contained in the transposon vector and excises the DNA fragment containing the exogenous gene sandwiched between the terminal inverted repeats from the transposon vector.

In order to select a target cell in which the exogeneous gene has been incorporated into the target site, it is preferable that the transposon vector contains a drug resistance gene or a reporter gene in the DNA fragment containing the exogenous gene. Here, the drug resistance gene and the reporter gene may be the same as those used in the targeting vector for gene introduction.

It is preferable that the drug resistance and reporter gene are under the control of any promoter that can function in the target cell. Here, the promoter may be the same as that used in the targeting vector for gene introduction.

The transposon vector preferably has a polyA signal downstream of the drug resistance or reporter gene, and may be the same as that used in the targeting vector for gene introduction.

In addition, the transposase expression vector may contain a drug resistance gene, a reporter gene, a promoter, a polyA signal, and the like, in addition to the gene encoding the transposase. The drug resistance gene, reporter gene, promoter, and polyA signal may be the same as those contained in the transposon vector.

The transposon vector and transposase expression vector may be introduced into the target cell by the same method as that used for the targeting vector for gene introduction.

Cells incorporating an exogeneous gene in the target site may be selected by the same method as the method for selecting homologous recombinant cells incorporating a targeting vector for gene introduction.

The DNA fragment of the exogeneous gene introduced as described above can be incorporated into the transposase target sequence TTAA of the genome of the target cell by using the transposon vector incorporating the DNA fragment of the exogeneous gene and the transposase expression vector. Unlike the homologous recombination method using the above-mentioned targeting vector for gene introduction, this method does not limit the site of integration of the exogeneous gene because the target sequence is TTAA. However, it is possible to remove the exogeneous gene incorporated into the genome without leaving any trace by subsequent expression of transposase.

The human T cells of the present invention can be obtained as described above. As described above, when the human T cells of the present invention are used as a source of transplant cells for T cell transplantation therapy, they can avoid GVHD and immune responses on the recipient side after transplantation. In addition, since the human T cells of the present invention are mostly the most undifferentiated naive T cells or stem cell-like memory T cells, they can survive stably for a long time after transplantation and exhibit therapeutic effects.

The human T cells of the present invention may also further contain a suicide gene. In particular, when the source of human T cells is a pluripotent stem cell such as a human iPS cell, it is desirable to introduce a suicide gene to avoid the risk of tumorigenicity of the obtained human T cells of the present invention. This makes it possible to remove only the human T cells of the present invention when the human T cells of the present invention cause undesirable side effects such as canceration after transplantation.

The suicide gene introduced into the human T cells of the present invention includes, but is not limited to, the HSV-tk gene and mutants thereof (e.g., HSV-TK, HSV-TK39, etc.) and iCaspase9 (e.g., AP1903-binding type, Rapamycin-binding type, etc.). A specific means for introducing the suicide gene may be the same as the means for introducing the exogenous gene described above. However, the expression of the suicide gene introduced into the human T cells of the present invention is manipulated freely. Therefore, in the suicide gene introduction targeting vector for introducing the suicide gene, the suicide gene is linked to a conditional promoter. An example of the conditional promoter is a promoter containing a Tet operator DNA sequence (tetO). A promoter containing the Tet operator DNA sequence (tetO) is driven by a complex of the reverse tetracycline-controlled transactivator (rtTA) and doxycycline (Dox).

The present invention also provides a method for producing a low-immunogenic human T cell, including
(1) a step of providing a human T cell having the following characteristics (a) and/or (b),
   (a) CD62L-positive and CD45RA-positive
   (b) a total ATP production rate of a human T cell-containing cell population of 400 pmol/min/10⁵ cells or more and a mitochondrial spare respiratory capacity of 40 pmol/min/10⁵ cells or more, 5-6 days after the application of T cell proliferation stimulation to the cell population
(2) a step of deleting an HLA class I molecule in the human T cell or a progenitor cell thereof, and
(3) a step of introducing exogeneous ST6GALNAC6 gene into the human T cell or a progenitor cell thereof (hereinafter also to be referred to as "the production method of the human T cell of the present invention").

In the method for producing human T cells of the present invention, each step can be performed by each method described above. Steps (2) and (3) can be performed in either order, or can be performed simultaneously. When steps (2) and (3) are performed on progenitor cells, step (1) is a step of differentiating the progenitor cells into CD62L-positive CD45RA-positive human T cells or equivalent human T cells.

Low-immunogenic human cells obtained as described above avoid immune responses and maintain the characteristics of the parent human cells, similar to the human T cells of the present invention. For example, when the parent human cells are human pluripotent stem cells, the obtained low-immunogenic human cells maintain the expression levels of undifferentiated markers, similar to the parent human pluripotent stem cells, have very similar overall gene expression patterns, and maintain the ability to differentiate into various cells.

The method for producing human T cells of the present invention may further include (4) a step of introducing a suicide gene into CD62L-positive CD45RA-positive human T cells or equivalent human T cells or progenitor cells thereof. In the method for producing human T cells of the present invention, step (4) can be performed by the above-mentioned method.

The human T cells of the present invention further introduced with a suicide gene obtained in this manner can be removed alone when undesirable side effects such as canceration occur after transplantation.

The low-immunogenic human T cells obtained as described above can be stably stored while maintaining long-term viability by cryopreserving them in a conventional manner until the time of producing genetically modified human T cells such as CAR-T cells or TCR-T cells.

The present invention also provides the use of the aforementioned human T cells of the present invention or the low-immunogenic human T cells obtained by the aforementioned method for producing human T cells of the present invention, for the production of genetically-modified human T cells for adoptive immunotherapy such as CAR-T cells or TCR-T cells. Another aspect of the present invention provides the use of the aforementioned human T cells having the ability to be maintained for a long time in vitro and/or the ability to remain in the body for a long time after transplantation, for the production of such low-immunogenic human T cells.

The human T cells of the present invention can be used not only as a source for the production of CAR-T cells or TCR-T cells, but also can be formulated as a transplantation therapy agent (TIL infusion therapy agent) as T cells expressing TCR against a cancer antigen by using tumor infiltrating lymphocytes (TIL) isolated from a donor expressing a target cancer antigen, without introducing a nucleic acid encoding CAR or exogenous TCR, and can be allogeneically transplanted into a patient expressing the same cancer antigen.

The human T cells having the ability to remain in the body for a long period and selected by the present invention can be used, after genetic modification to be low immunogenic, as a source for the production of the above-mentioned CAR-T cell or TCR-T cell therapy agent, or by itself as a transplantation therapy agent for allogeneic transplantation. Furthermore, the human T cells can be used as a source for producing CAR-T cells or TCR-T cells for autologous transplantation or allogeneic transplantation with the same HLA (e.g., HLA-A, HLA-B, HLA-DR). Alternatively, the human T cells themselves can be used as a transplantation therapy agent for autologous transplantation or allogeneic transplantation with the same HLA (e.g., HLA-A, HLA-B, HLA-DR), by using tumor-infiltrating lymphocyte (TIL) isolated from a donor (including the patient himself) expressing the target cancer antigen.

For producing CAR-T cells or TCR-T cells from the human T cells of the present invention or human T cells to be used in the present invention, which have the ability to be maintained for a long time in vitro and/or the ability to remain in the body for a long time after transplantation (hereinafter sometimes to be comprehensively referred to as "the T cell of the present invention"), a method known per se can be appropriately selected and used.

### (a) Nucleic acid encoding CAR

CAR is an artificially constructed hybrid protein that includes an antigen-binding domain of an antibody **(e.g.,** scFv) linked to a T cell signal transduction domain. The features of CAR include the ability to utilize the antigen-binding properties of monoclonal antibodies to redirect the specificity and reactivity of T cells to a selected target in a non-MHC-restricted manner. Non-MHC-restricted antigen recognition confers T cells expressing the CAR with the ability to recognize antigens independent of antigen processing, thereby circumventing a major mechanism of tumor escape. Furthermore, when expressed in T cells, the CAR advantageously does not dimerize with endogenous TCR α and β chains.

The CAR introduced into the human T cells of the present invention includes an antibody antigen-binding domain that can specifically recognize the surface antigen (e.g., cancer antigen peptide, surface receptors whose expression is enhanced in cancer cells, etc.) to be recognized by the target T cell, an extracellular hinge domain, a transmembrane domain, and an intracellular T cell signal transduction domain.

Examples of the surface antigen specifically recognized by antigen binding domain include, but are not limited to, surface receptors with promoted expression in various cancers (e.g., acute lymphocytic carcinoma, alveolar rhabdomyosarcoma, bladder cancer, bone cancer, brain cancer (e.g., medulloblastoma), breast cancer, anal, anal canal, or anorectal cancer, eye cancer, intrahepatic bile duct cancer, joint cancer, neck, gallbladder, or pleural cancer, nose, nasal cavity, or middle ear cancer, oral cancer, vulva cancer, chronic myeloid cancer, colon cancer, esophageal cancer, cervical cancer, fibrosarcoma, gastrointestinal carcinoid tumor, head and neck cancer (e.g., head and neck squamous cell carcinoma), hypopharyngeal cancer, kidney cancer, laryngeal cancer, leukemia **(e.g.,** acute lymphoblastic leukemia, acute lymphocytic leukemia, chronic lymphocytic leukemia, acute myeloid leukemia), liquid tumors, liver cancer, lung cancer **(e.g.,** non-small cell lung cancer), lymphoma **(e.g.,** Hodgkin's lymphoma, non-Hodgkin's lymphoma, diffuse large B-cell lymphoma, follicular lymphoma), malignant mesothelioma, mast cell tumor, melanoma, multiple myeloma, nasopharyngeal carcinoma, ovarian cancer, pancreatic cancer; peritoneal, omental and mesenteric cancer; pharyngeal cancer, prostate cancer, rectal cancer, renal cancer, skin cancer, small intestine cancer, soft tissue cancer, solid tumors, gastric cancer, testicular cancer, thyroid cancer, ureteral cancer, and the like), for example, CD19, EGF receptor, BCMA, CD30, Her2, ROR1, MUC16, CD20, mesothelin, B-cell mutation antiten, CD123, CD3, prostate specific membrane antigen (PSMA), CD33, MUC-1, CD138, CD22, GD2, PD-L1, CEA, chondroitin sulfate proteoglycan-4, IL-13 receptor α chain, IgGκ light chain and the like, cancer antigen peptide (e.g., peptides derived from WT1, GPC3, MART-1, gp100, NY-ESO-1, MAGE-A4, etc.), and the like.

The antigen-binding domain used in the present invention is not particularly limited as long as it is an antibody fragment capable of specifically recognizing a target antigen. Considering the ease of CAR production, it is preferable to use a single-chain antibody (scFv) in which a light chain variable region and a heavy chain variable region are linked via a linker peptide. The arrangement of the light chain variable region and the heavy chain variable region in a single-chain antibody is not particularly limited as long as both can reconstitute a functional antigen-binding domain. They are generally designed in the order of light chain variable region-linker peptide-heavy chain variable region, from the N-terminus. As the linker peptide, a linker peptide known per se that is generally used in the production of single-chain antibodies can be used. The DNA encoding the light chain variable region and the DNA encoding the heavy chain variable region can be prepared, for example, by cloning the light chain gene and the heavy chain gene from antibody-producing cells, respectively, and performing PCR using them as templates, or can be chemically synthesized from the sequence information of an existing antibody. The obtained DNA fragments and the DNA encoding the linker peptide are ligated by an appropriate method to obtain DNA encoding a single-chain antibody. It is preferable that a leader sequence is further added to the N-terminus of the antigen-binding domain in order to present the CAR on the T cell surface.

As the extracellular hinge domain and transmembrane domain, a domain derived from a T cell surface molecule generally used in the technique field can be appropriately used, and examples thereof include, but are not limited to, each domain derived from CD8α or CD28.

As the intracellular signal transduction domain, examples include, but are not limited to, those having a CD3ζ chain, those having a costimulatory signal transduction motif such as CD28, CD134, CD137, Lck, DAP10, ICOS, or 4-1BB between the transmembrane domain and the CD3ζ chain, and those having two or more costimulatory signal transduction motifs, and any domain commonly used in the technique field can be used in combination.

Nucleic acid sequence information encoding the extracellular hinge domain, transmembrane domain, and intracellular signal transduction domain is well known in the art, and a person skilled in the art can easily obtain, based on the information, DNA fragments encoding each domain from T cells.

The DNA fragments thus obtained, each encoding the antigen-binding domain, the extracellular hinge domain, the transmembrane domain, and the intracellular signal transduction domain, can be linked by a conventional method to obtain DNA encoding CAR.

The obtained DNA encoding CAR can be inserted into an expression vector, preferably a plasmid vector, containing a promoter functional in T cells, either as is or after the addition of an appropriate linker and/or a nuclear localization signal, and the like. Examples of the promoter functional in T cells include, but are not limited to, the constitutive SRα promoter, SV40 promoter, LTR promoter, CMV (cytomegalovirus) promoter, RSV (Rous sarcoma virus) promoter, MoMuLV (moloney murine leukemia virus) LTR, and HSV-TK (herpes simplex virus thymidine kinase) promoter, which are constitutive in mammalian cells. Gene promoters such as CD3, CD4, and CD8 that are specifically expressed in T cells can also be used.

### (b) Nucleic acid encoding exogenous TCR

In this specification, "T cell receptor (TCR)" means a receptor composed of a dimer of TCR chains (α chain, β chain) that recognizes an antigen or the antigen-HLA complex and transmits a stimulatory signal to a T cell. Each TCR chain is composed of a variable region and a constant region, and the variable region has three complementarity determining regions (CDR1, CDR2, CDR3). The TCR used in the present invention includes not only TCR in which the α chain and β chain of TCR constitute a heterodimer, but also TCR in which the α chain and β chain constitute a homodimer. Furthermore, the TCR includes TCR in which a part or all of the constant region is deleted, TCR in which the amino acid sequence is modified, and TCR in which the constant region is soluble.

In addition, "exogenous TCR" means that it is exogenous to T cell as the target cell. The amino acid sequence of the exogenous TCR may be the same as or different from the endogenous TCR expressed by T cell as the target cell.

The nucleic acid encoding TCR is a nucleic acid encoding the α-chain and β-chain of TCR capable of specifically recognizing a surface antigen (e.g., cancer antigen peptide, etc.) to be recognized by the target T cell.

The nucleic acid can be prepared by a method known per se. When the amino acid sequence or nucleic acid sequence of the desired TCR is known, it is possible to construct a DNA encoding the entire length or a part of the TCR of the present invention based on the sequence, for example, by chemically synthesizing a DNA chain or an RNA chain, or by connecting synthesized short overlapping oligo-DNA chains by using the PCR method or the Gibson Assembly method.

When the sequence of the target TCR is not known, for example, the target T cell can be isolated from a cell population containing T cells expressing the target TCR, and the nucleic acid encoding the TCR can be obtained from the T cell. Specifically, a cell population containing T cells (e.g., PBMC) is collected from a human, and the cell population is stimulated and cultured in the presence of an epitope of a cell surface antigen recognized by a target TCR. From the cell population, T cells that specifically recognize cells expressing the cell surface antigen can be selected by a known method using the specificity for cells expressing the cell surface antigen and cell surface antigens such as CD8 and CD4 as indicators. The specificity of T cells for cells expressing the surface antigen can be measured, for example, by using dextromer assay, ELISPOT assay, or cytotoxicity assay. The cell population containing T cells is preferably collected, for example, from a living body having a large number of cells expressing a cell surface antigen recognized by the desired TCR (e.g., a patient with a disease such as cancer, or a T cell-containing population that has been contacted with an epitope of the antigen or dendritic cells pulsed with the epitope).

The nucleic acid of the present invention can be obtained by extracting DNA from the isolated T cells by a conventional method, amplifying the TCR gene based on the nucleic acid sequence of the constant region of the TCR using the DNA as a template, and cloning same. Alternatively, RNA is extracted from the cells by a conventional method to synthesize cDNA, and then using the cDNA as a template, 5'-RACE (Rapid amplification of cDNA ends) is performed using antisense primers complementary to the nucleic acids encoding the constant regions of the TCR α and β chains. 5'-RACE can be performed by a known method, for example, using a commercially available kit such as SMART PCR cDNA Synthesis Kit (Clontech). The obtained DNAs encoding the α and β chains of TCR can be inserted into an appropriate expression vector, similar to the DNA encoding the aforementioned CAR. The DNA encoding the α chain and the DNA encoding the β chain may be inserted into the same vector or into separate vectors. When inserted into the same vector, the expression vector may express both chains polycistronically or monocistronically. In the former case, an intervening sequence that allows polycistronic expression, such as IRES or FMV 2A, is inserted between the DNAs encoding both chains.

The method for introducing the nucleic acid encoding the above-mentioned CAR or exogenous TCR or the vector containing same into cells is not particularly limited, and any known method can be used. When a nucleic acid or a plasmid vector is introduced, for example, calcium phosphate coprecipitation method, PEG method, electroporation method, microinjection method, lipofection method, and the like can be used. For example, the methods described in Cell Engineering Special Issue 8: New Cell Engineering Experimental Protocols, 263-267

(1995) (published by Shujunsha), Virology, Vol. 52, 456 (1973), Folia Pharmacol. Jpn., Vol. 119 (No. 6), 345-351 (2002), and the like can be used. When a viral vector is used, the nucleic acid can be introduced into cells by introducing the nucleic acid into a suitable packaging cell (e.g., Plat-E cell) or a complementation cell line (e.g., 293 cell), recovering the viral vector produced in the culture supernatant, and infecting the cells with the vector by a method appropriate for each viral vector. For example, a specific means using a retroviral vector as a vector are disclosed in International Publication No. 2007/69666, Cell, 126, 663-676 (2006), and Cell, 131, 861-872 (2007), and the like. In addition, a specific means using a lentivirus as a vector are disclosed in Zufferey R. et al., Nat Biotechnol, 15(9):871-895 (1997), and the like. In particular, when a retroviral vector is used, highly efficient gene transfer into various cells is possible by using a recombinant fibronectin fragment, CH-296 (manufactured by Takara Bio Inc.). Alternatively, the nucleic acid or vector of the present invention may be introduced into the genome of a cell by genome editing (e.g., CRISPR system, TALEN, ZFN, etc.).

The CAR-T or TCR-T cells obtained as described above can specifically recognize and kill (e.g., induce apoptosis) cells expressing a surface antigen specifically recognized by the CAR or exogenous TCR expressed by the T cells. Therefore, human T cells delivered with a nucleic acid encoding a CAR or exogenous TCR that recognizes a surface molecule that is specifically expressed or is enhanced in diseased cells such as cancer cells as a surface antigen can be used for the prevention or treatment of diseases such as cancer, and can be safely administered to humans.

In a medicament containing the CAR-T or TCR-T cells as an active ingredient, the T cells may be cultured using an appropriate medium before administration to a subject. In addition, a stimulatory molecule may be added to the medium to maintain and amplify the activation and/or proliferation of the T cells. Furthermore, serum or plasma may be added to the medium. The amount of these to be added to the medium is not particularly limited, and 0% to 20% by volume is exemplified, and the amount of serum or plasma to be used can be changed depending on the culture stage. For example, the serum or plasma concentration can also be gradually reduced. The serum or plasma may be derived from either autologous or non-autologous sources, but from the aspect of safety, autologous sources are preferred.

The medicament containing the CAR-T or TCR-T cell as an active ingredient is preferably administered parenterally to the subject. Parenteral administration method includes intravenous, intraarterial, intramuscular, intraperitoneal, and subcutaneous administrations. The dosage is appropriately selected according to the condition, weight, age, and the like of the subject. Generally, the number of cells is generally 1×10⁶ to 1×10¹⁰, preferably 1×10⁷ to 1×10⁹, more preferably 5×10⁷ to 5×10⁸, per administration to a subject weighing 60 kg. In addition, it may be administered once or multiple times. The medicament may be in a known form suitable for parenteral administration, such as an injection or infusion. The medicament may contain a pharmacologically acceptable excipient as appropriate. The medicament may contain saline, phosphate buffered saline (PBS), culture medium, and the like, in order to stably maintain the cells. Examples of the culture medium include, but are not limited to, RPMI, AIM-V, X-VIVO10, and the like. In addition, the medicament may contain a pharmaceutically acceptable carrier (e.g., human serum albumin), a preservative, and the like, for the purpose of stabilization.

The medicament containing the CAR-T or TCR-T cell as an active ingredient can be a prophylactic or therapeutic drug for cancer. The cancer to which the medicament is applied is not particularly limited and examples thereof include, but are not limited to, acute lymphocytic cancer, alveolar rhabdomyosarcoma, bladder cancer, bone cancer, brain cancer (e.g., medulloblastoma), breast cancer, anal, anal canal, or anorectal cancer, eye cancer, intrahepatic bile duct cancer, joint cancer, neck, gallbladder, or pleural cancer, nose, nasal cavity, or middle ear cancer, oral cancer, vulva cancer, chronic myeloid cancer, colon cancer, esophageal cancer, cervical cancer, fibrosarcoma, gastrointestinal carcinoid tumor, head and neck cancer (e.g., head and neck squamous cell carcinoma), hypopharyngeal cancer, kidney cancer, laryngeal cancer, leukemia (e.g., acute lymphoblastic leukemia, acute lymphocytic leukemia, chronic lymphocytic leukemia, acute myeloid leukemia), liquid tumors, liver cancer, lung cancer (e.g., non-small cell lung cancer), lymphoma (e.g., Hodgkin's lymphoma, non-Hodgkin's lymphoma, diffuse large B-cell lymphoma, follicular lymphoma), malignant mesothelioma, mast cell tumor, melanoma, multiple myeloma, nasopharyngeal cancer, ovarian cancer, pancreatic cancer; peritoneal, omental and mesenteric cancer; pharyngeal cancer, prostate cancer, rectal cancer, renal cancer, skin cancer, small intestine cancer, soft tissue cancer, solid tumor, stomach cancer, testicular cancer, thyroid cancer, ureteral cancer, and the like.

The present disclosure is described in more detail in the following with reference to examples. These are merely illustrative and the present disclosure is not limited to these examples.

### [Example]

### Example 1: Isolation and amplification of CD62L⁺CD45RA⁺ cells (1)

To overcome the technical problem of enabling stable long-term retention in the body after transplantation, a cell culture system that enables the isolation, amplification, cryopreservation, and long-term maintenance culture of CD62L⁺CD45RA⁺ cells from human peripheral blood was established.

**[Table 1]**

| Production of PBMC-derived T cells | | | | |
|---|---|---|---|---|
| Material name | Manufacturer | Model number | Use | Notes |
| PBMC | | | | |
| Human AB Serum, Off the clot | Valley Biomedical | HS1017 | serum | |
| KBM501 | KOJIN | 16025015 | Primary medium | IL-2 1750 JRU/mL added |
| KBM502 | KOJIN | 16025020 | Additional medium for expansion | IL-2 175 JRU/mL added |
| T Cell TransAct, Human | Miltenyi Biotec | 130-111-160 | T lymphocyte-activating antibody | |
| T225 flask | | | | for adherent cells |

**[Table 2]**

| | | |
|---|---|---|
| antibody for FACS | | |

| **Product name** | **Manufacturer** | Cat No. |
|---|---|---|
| BV421 Mouse Anti-Human CD3 | BD Biosciences | 563798 |
| PE Mouse Anti-Human CD62L | BD Biosciences | 555544 |
| PE-Cy7 Mouse Anti-Human CD45RA | BD Biosciences | 560675 |
| FITC Mouse Anti-Human CD45R0 | BD Biosciences | 555492 |
| APC Mouse Anti-Human CCR7 (CD197) | BD Biosciences | 566762 |

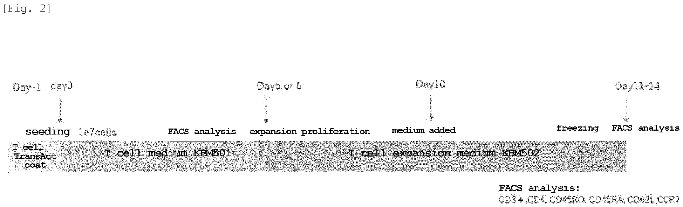

The culture protocol is schematically shown in Fig. 2. On the day before the start of isolation, T Cell TransAct was adjusted to 5 µg/15 ml PBS, placed in a T225 flask, and left standing at 4°C overnight or longer. Frozen PBMC was thawed in KBM501 medium, and the cell number was counted. The flask containing the T cell activating antibody was removed from the refrigerator, the antibody solution was removed by suction, 20 mL of new PBS was added to the flask, and the bottom was slowly shaken and then suctioned, which was repeated twice. Culture medium was added to 1.3x10^7 cells/20 mL KBM501, AB Serum was added to a 10% amount, and the cells were cultured in an incubator under the conditions of 37°C, 5% CO₂. The day of seeding was set as day 0, and on day 3, the cells were examined under a microscope. When 3-5 clusters of 6-10 cells were confirmed in one field of view (x40), the cells were diluted to 100 mL with KBM501. On day 6, the medium turned yellow, and the cell number increased to 2.7x10^8 cells/100 mL. 1.5x10^8 cells were dispensed into 10 tubes at 1.5x10^7 cells/tube and cryopreserved. The remaining 1.3x10^8 cells were dispensed into two flasks at 6.5x10^7 cells/50 mL/T225 flask and expansion cultured. On day 9, the flasks were examined under a microscope, and when 3-5 clusters of 6-10 cells were confirmed in one field of view (x40), the flasks were filled up to 100 mL with KBM502. After FACS analysis of the T cells was performed around day 10, the cells were frozen at 1x10^8 cells/mL. On days 11-14, the frozen cells were awakened and FACS analysis was performed.

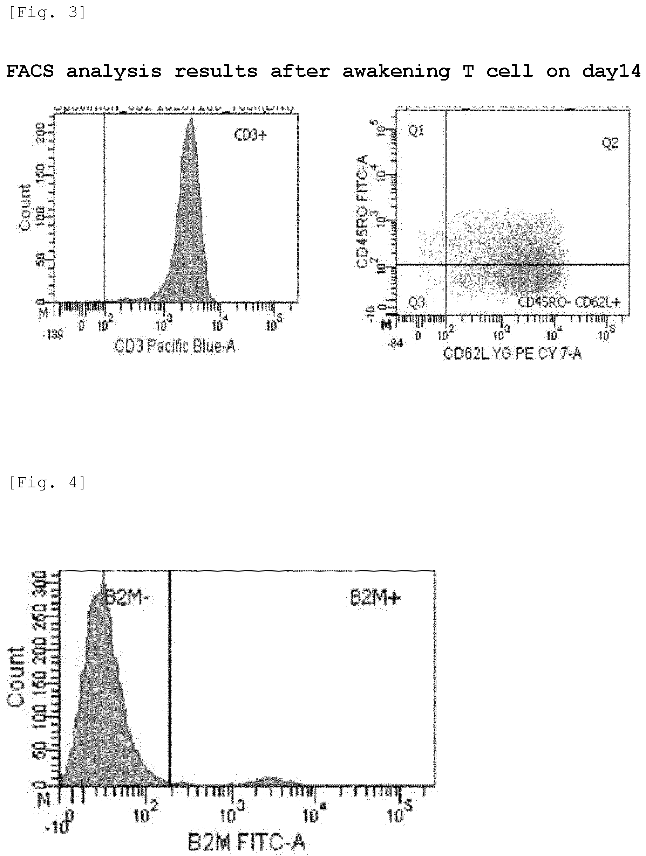

The results are shown in Fig. 3. A large amount of human T cell fraction that was CD3-positive, CD62L-positive, and CD45RO-negative (presumably CD45RA-positive) could be obtained.

### Example 2: Production of B2M gene knockout/ST6GALNAC6 gene-transduced human T cells (1)

To produce low-immunogenic (universal donor) human T cells that are not restricted by HLA antigens, the B2M gene was knocked out (KO) using CRISPR-Cas9 gene editing technology in the CD62L⁺CD45RA⁺ cells isolated in Example 1, whereby all HLA class I molecules (A, B, C, E, G, F) were deleted.

Furthermore, since HLA class I KO cells are attacked by NK cells as non-self, attempts were made to avoid this by suppressing the activation of NK cells by inducing the activity of the ITIMS motif in NK cells. Specifically, the ST6GALNAC6 gene, which encodes DSGb5 that specifically binds to the NK cell receptor Siglec-7 (CD328), was forcibly expressed in B2M KO, CD62L-positive T cells, and immune tolerance was induced by inducing the activity of the intracellular ITIMS motif of CD328 in NK cells.

**[Table 3]**

| **Product name** | Gene | **Manufacturer** | gRNA ID | Target DNA Sequence | PAM Sequence | Target locus |
|---|---|---|---|---|---|---|
| Invitrogen TrueGuide^{™} sgRNA Modified | B2M | Invitrogen | CRISPR98370 7 SGM | AGTCACATGGT TCACACGGC | AGG | Chr. 15: 44715651 - 44715673 on GRCh38 |
| Invitrogen^{™} TrucCut^{™} Cas9 Protein v2 (5mg/mL) | | Invitrogen | | | | |
| P3 Primary Cell 4D-Nucleofecto r^{™} X Kit | | LONZA | | | | |
| ST6GALNAC6. Lenti ORF particles | ST6GALNAC6 | ORIGENE | | | | |

KBM501+10% AB serum was added to a 50 ml tube and allowed to return to room temperature. The cell freezing tube was removed from the liquid nitrogen tank and rapidly thawed by warming in a thermostatic tank (heat block) at 37°C for 60 to 90 seconds. Warming was stopped when only a small amount of ice remained. 3 ml of the above-mentioned medium was quickly aspirated with a pipette, and the contents of the tube containing the cells under thawing were also aspirated and pipetted to completely thaw the frozen cells, which were then collected in a 14 ml tube. The tube was centrifuged at 1600 rpm for 5 minutes, and the supernatant was removed. The pellet was completely broken by tapping 2-3 times, 5 mL of medium was added, and the cells were dispersed by pipetting 2-3 times. The number of cells was then counted using trypan blue. The cells were suspended in 20 ml of KBM501 to 2-3x10^7 cells (1-2x10^6/mL), placed in a flask, and cultured at 37°C in a CO₂ incubator. The next day, microscopic examination confirmed that multiple large clusters (2-3 per field) had been formed, and 2.5-fold amount of KBM501 medium was added. The next day, KBM502 was added to 5x10^5 cells/mL, and counting and subculture were performed once every 3 days thereafter. FACS was performed the next day and thereafter. 5x10^6 T cells from day 2 after awakening were centrifuged, the medium was removed, and the cells were rinsed once with PBS. Cas9 17.5 ug (3.5 uL), sgRNA B2M 7.5 uL (50 uM), and P3 Kit solution 88 uL were mixed with the above-mentioned cell pellets and sgRNA was introduced using the EO-115 program of AMAXA4D. Cells were cultured at 5x10^5/mL in KBM502. Five days after introduction, B2M was confirmed to have been knocked out by FACS (Fig. 4). 95% of the cells were knocked out. Then, 5x10^5 cells were infected with 3 TU of ST6GALNAC6. Lenti. After four days after infection (more than twice the proliferation compared to before infection), the cells were co-cultured with NK cells to confirm the establishment of immune tolerance.

### Experimental Example 1: Induction of immune tolerance to NK cell (1)

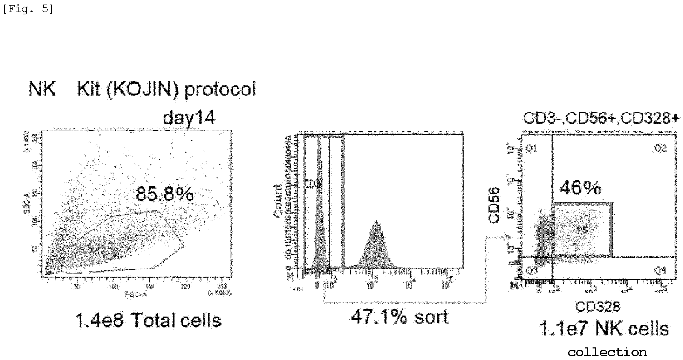

Human NK cells were isolated using the KBM NK kit (NK cell selection and culture kit) (KOHJIN BIO) according to the manufacturer's protocol (Fig. 5). Using the following reagents and the like, the genetically modified human T cells obtained in Example 2 and human NK cells were co-cultured for 24 hours at a ratio of T cell:NK cell = 1.7:1. KBM502 was used as the medium. For comparison, B2M KO T cells without introduction of ST6GALNAC6 gene (mock) were also tested in the same manner.

**[Table 4]**

| **Product name** | **Manufacturer** | Cat No. |
|---|---|---|
| APC Mouse Anti-Human CD56 | BD Biosciences | 555518 |
| CD328 (Siglec7) Monoclonal Antibody, PE, eBioscience | invitrogen | 12-5759-42 |
| PE-Cy7 Mouse Anti-Human CD62L | BD Biosciences | 565535 |
| FITC Mouse Anti-Human *β* 2-Microglobuln | BD Biosciences | 551338 |

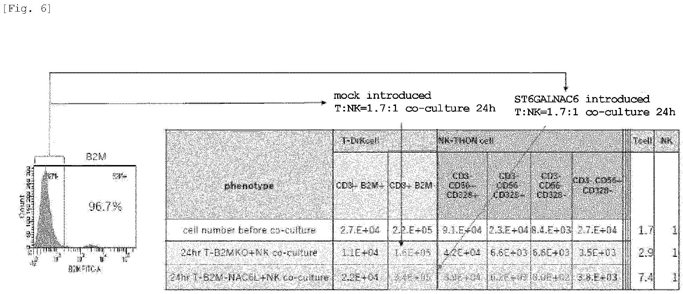

After counting the total cells, the CD3 percentage was confirmed by FACS, and changes in the ratio of T cell:NK cell were examined. As a result, it was confirmed that attacks from NK cells were highly suppressed in T cells introduced with the ST6GALNAC6 gene (Fig. 6).

### Example 3: Isolation and amplification of CD62L+CD45RA+ cells (2)

A culture method that can more efficiently isolate and expand CD62L+CD45RA+ cells from human peripheral blood was investigated.

**[Table 5]**

| Production of PBMC-derived T cells | | | | |
|---|---|---|---|---|
| Material name | Manufacturer | Model number | Use | Notes |
| PBMC | | | | |
| Human AB Serum, Off the clot | Valley Biomedical | HS1017 | serum | |
| KBM501 | KOJIN | 16025015 | Primary medium | IL-2 1750 JRU/mL added |
| KBM502 | KOJIN | 16025020 | Additional medium for expansion | IL-2 175 JRU/mL added |
| T Cell TransAct, Human | Miltenyi Biotec | 130-111-160 | T lymphocyte-activating antibody | |
| Container for suspended cells | | | T lymphocyte culture | 6 well plate or T flask |

**[Table 6]**

| | | |
|---|---|---|
| FACS antibody | | |

| **Product name** | **Manufacturer** | Cat No. |
|---|---|---|
| BV421 Mouse Anti-Human CD3 | BD Biosciences | 563798 |
| APC Mouse Anti-Human CD4 | BD Biosciences | 561841 |
| FITC Mouse Anti-Human CD8 | BD Biosciences | 560960 |
| PE Mouse Anti-Human CD62L | BD Biosciences | 555544 |
| PE-Cy7 Mouse Anti-Human CD45RA | BD Biosciences | 560675 |
| FITC Mouse Anti-Human CD45RO | BD Biosciences | 555492 |
| APC Mouse Anti-Human CCR7 (CD197) | BD Biosciences | 566762 |
| 7AAD | BD Biosciences | 51-68981E |

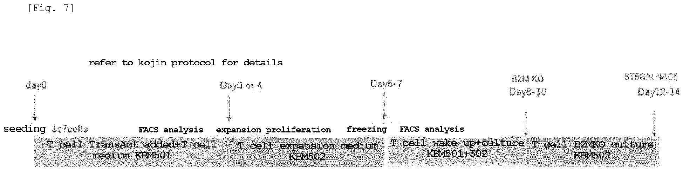

Improved culture protocol is schematically shown in Fig. 7. Frozen PBMC was thawed in KBM501 medium, the cell number was counted, and KBM501 medium was added to 5x10^6 cells/5 mL. After further addition of AB Serum to a volume of 10%, the cells were cultured in an incubator under the conditions of 37°C, 5% CO₂. The day of seeding was designated day 0, the cells were centrifuged on day 2, 5 mL of KBM501 medium was added, and the cells were further cultured. On day 4, the cells were counted and centrifuged, and adjusted to 5x10^5 cells/mL with KBM502 medium. The cells were counted on days 5 and 6, and when the cells proliferated about 100-fold from day 0, they were stocked by 1x10^7 cells/vial (5x10^6 cells/mL) and cryopreserved.

Frozen stock cells were thawed in KBM501 medium, the number of cells was counted, then KBM501 medium was added to 1x10^6 cells/mL, and AB Serum was added to a 10% amount. The cells were cultured in an incubator under the conditions of 37°C, 5% CO₂ for 2 days, after which the B2M gene was knocked out (see Example 4). Then, KBM502 medium was added to the cells, and the cells were cultured in an incubator under the conditions of 37°C, 5% CO₂ for additional 3 to 4 days. FACS analysis was performed on day 12.

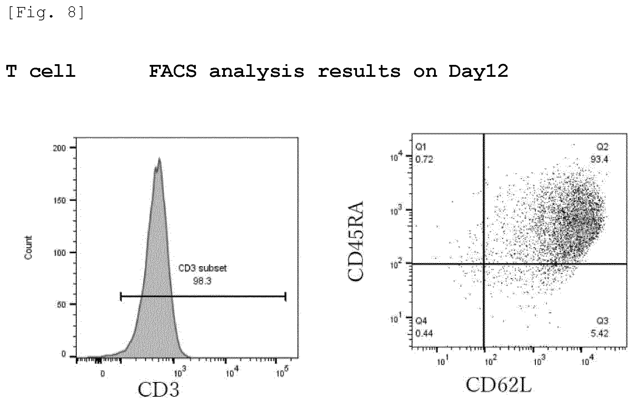

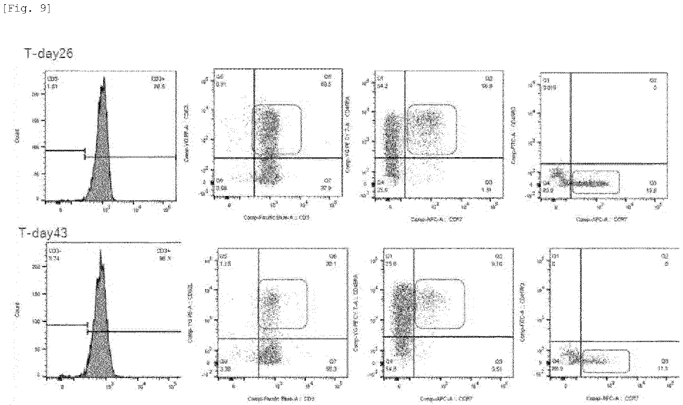

The results are shown in Fig. 8. A large amount of human T cell fraction that was CD3-positive, CD62L-positive, and CD45RA-positive could be obtained. The cells obtained in this Example and Example 1 were further cultured in KBM502 medium, and FACS analysis was performed on day 26 and day 43, respectively. The results are shown in Fig. 9. It was clarified that the T cell population obtained in this Example had a higher proportion of CD62L-positive, CD45RA-positive, CCR7-positive, and CD45RO-negative cells (naive T cells or stem cell-like memory T cells) than the T cell population obtained in Example 1.

### Example 4: Production of B2M gene knockout/ST6GALNAC6 gene-transduced human T cells (2)

As in Example 2, the B2M gene was knocked out and the ST6GALNAC6 gene was introduced into the human T cells of Example 3 by using the reagents and kits shown in Table 3.

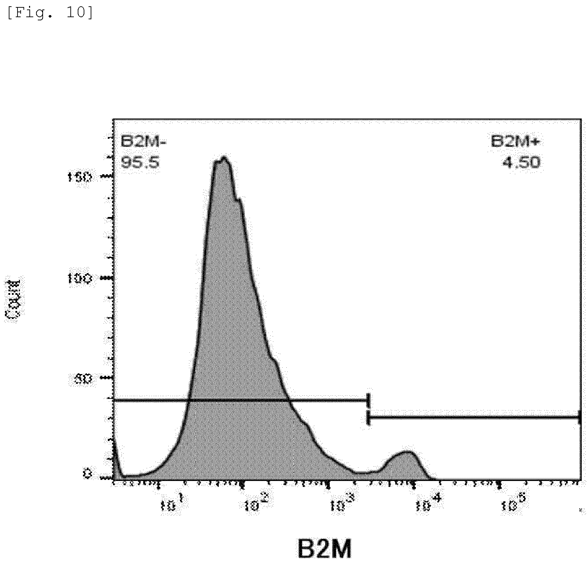

KBM501+10% AB serum was added to a 14 ml tube and allowed to return to room temperature. The cell freezing tube was removed from the liquid nitrogen tank and rapidly thawed by warming in a thermostatic tank (heat block) at 37°C for 60 to 90 seconds. 3 ml of the above-mentioned medium was quickly aspirated with a pipette, and the contents of the tube containing the cells under thawing were also aspirated and pipetted to completely thaw the frozen cells, which were then collected in a 14 ml tube. The tube was centrifuged at 1500 rpm for 5 minutes, and the supernatant was removed. 5 mL of medium was added, and the cells were dispersed by pipetting 2-3 times. The number of cells was then counted using trypan blue. The cells were suspended in KBM501+10% AB serum to 2x10^6/mL, and cultured at 37°C in a CO₂ incubator. The next day, microscopic examination confirmed that multiple large clusters (2-3 per field) had been formed, and 2.5-fold amount of KBM501 medium was added. The next day, the cells were centrifuged, KBM502 was added, and the cell number was counted and adjusted to 5x10^5 cells/mL. 5x10^6 T cells from day 2 after awakening were centrifuged, the medium was removed, and the cells were rinsed once with PBS. Cas9 17.5 ug (3.5 uL), sgRNA B2M 7.5 uL (50 uM), and P3 Kit solution 88 uL were mixed with the above-mentioned cell pellets and sgRNA was introduced using the EO-115 program of AMAXA4D. Cells were cultured at 1x10^6/mL in KBM502. Five days after introduction, B2M was confirmed to have been knocked out by FACS (Fig. 10). 95% of the cells were knocked out. Then, 5x10^5 cells were infected with 3 TU of ST6GALNAC6. Lenti. After four days after infection (more than twice the proliferation compared to before infection), the cells were co-cultured with NK cells to confirm the establishment of immune tolerance.

### Experimental Example 2: Induction of immune tolerance to NK cell (2)

### 1) Separation of NK cells

Using the materials shown in Table 7, human NK cells were isolated by the following method.

**[Table 7]**

| Material name | Manufacturer | Model number | Use | Notes |
|---|---|---|---|---|
| PBMC | | | | |
| Mononuclear Cell Medium | Promocell | C-28030 | | |
| Dynabeads^{™} CD3 | Thermo | 11151D | for CD3 removal | |
| 0.1% BSA+2 mM EDTA | | | | produced on demand |
| Human AB serum | Valley Biomedical | HS1017 | NK culture | |
| Human Plasma | KOJIN | | NK culture | immobilized |
| KBM NK kit | KOJIN | 16025015 | Primary medium | NKCC-C, NKCC-1 and 2 |
| Container for adherent and suspended cells | | | NK cell culture | T flask |

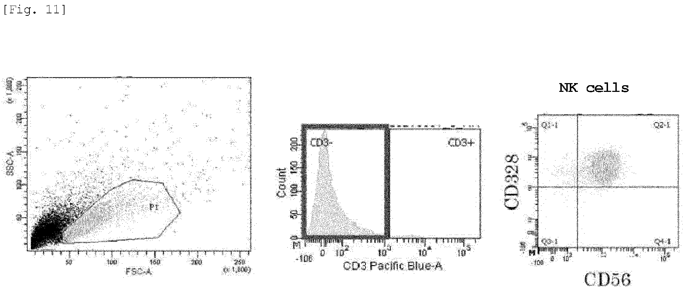

Human NK cells were isolated using the KBM NK kit (NK cell selection and culture kit) (KOHJIN BIO) according to the manufacturer's protocol with some modifications (Fig. 11).

### 2) Immune tolerance induction assay

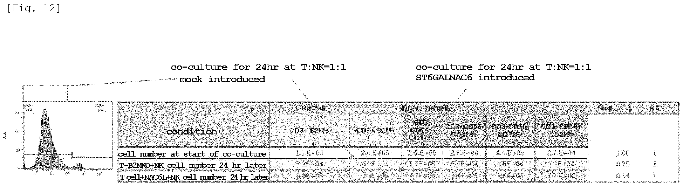

Using the reagents and the like shown in Table 4, the genetically modified human T cells obtained in Example 3 and human NK cells prepared in 1) were co-cultured for 24 hours at a ratio of T cell:NK cell = 1.7:1. KBM502 was used as the medium. For comparison, B2M KO T cells without introduction of ST6GALNAC6 gene (mock) were also tested in the same manner. After counting the total cells, the CD3 percentage was confirmed by FACS, and changes in the ratio of T cell:NK cell were examined. As a result, it was confirmed that attacks from NK cells were highly suppressed in T cells introduced with the ST6GALNAC6 gene (Fig. 12).

### Example 5: Identification of human T cells superior in long-term maintenance ability and long-term in vivo retention ability after transplantation using mitochondrial activity index

### 1) Isolation and expansion culture of human T cells

Using the materials shown in Table 8, human T cells were isolated and expansion cultured from human peripheral blood mononuclear cells (PBMC) by the following method. The background of the human PBMC donor is shown in Table 9.

**[Table 8]**

| Material name | Manufacturer | Model number | Use | Notes |
|---|---|---|---|---|
| human PBMC | | | separation, expansion culture of T cells | |
| Human AB Serum, Off the clot | Valley Biomedical | HS1017 | serum | |
| KBM502 | KOJIN | 16025020 | Additional medium for expansion | IL-2 175JRU/mL added |
| MACS^{®} GMP T Cell TransAct^{™} | Miltenyi Biotec | 170-076-156 | T lymphocyte-activating antibody | |
| CryoStor CS5 Bottle | Waken B-Tech | 205102 | Cryopreservation of T cells | |
| Container for floating cells | | | T lymphocyte culture | 6 well plate or T flask |

**[Table 9]**

| | Sex | Age | Ethnicity | Smoker (Yes or No) | BMI | **frozen T cells used** |
|---|---|---|---|---|---|---|
| Lot1 | Female | 51 | Caucasian | No | **41.78 (Obesity 4)** | Day 5,10 |
| Lot2 | Female | 45 | Caucasian | Yes | **25.2 (Obesity 1)** | Day6,10 |
| Lot3 | male | 67 | Asian | No | - | Day5,10 |
| Lot4 | male | 45 | Asian | Yes | - | Day 5,10 |

1-1) Frozen PBMCs were thawed in KBM502 medium and the cell density was counted. KBM502 medium was added to a cell density of 2x10^5/mL, AB Serum was added to a 10% amount, 57 µL of T Cell TransAct was simultaneously added, and culture was started in a 5% CO₂ incubator at 37°C (day 0). On day 3, the cells were centrifuged and 5 mL of KBM502 was added. On day 4, the cells were counted and centrifuged, and adjusted to 5x10^5/mL with KBM502 medium. On day 3, the cells were centrifuged to remove T Cell TransAct. On day 5 (some on day 6), the cells were counted and, after confirmation of about 30-fold proliferation from day 0, the freezing solution CryoStor CS5 was added to 1×10^7/mL, and the cells were frozen using the slow cooling method with Cryo5. The next day, the cell stock was transferred to liquid nitrogen. Some of the cells were cultured until day 10, and then frozen in the same manner as on day 5.

1-2) Frozen human T cells on day 5 were awakened in a 37°C heater, 1 mL of 10% AB serum-containing KBM502 medium was added to the freeze-thaw solution, and the cell suspension was transferred to a 15 mL tube containing 9 mL of 10% AB serum-containing KBM502 medium. After centrifugation at 1500 rpm for 5 minutes at room temperature, the supernatant was removed with an aspirator and the cells were loosened by tapping. KBM502 medium was added to 2×10^6/mL, AB Serum was added to a 10% amount, and culture was started. The next day, when clusters were formed, half the amount of the medium was added. After confirming that the cells were floating, they were counted and adjusted to 5x10^5/mL with KBM502 medium. Until day 10, a portion of the cells was counted once every two days, and medium was added without breaking the cell aggregates. Thereafter, a freezing solution was added to 1x10^7/mL, and the cells were frozen using the slow cooling method with Cryo5. The next day, the cell stock was transferred to liquid nitrogen.

### 2) Measurement of ATP production rate and mitochondrial spare respiratory capacity

Using the materials shown in Table 10, the ATP production rate and mitochondrial spare respiratory capacity of human T cells were measured by the method shown below.

**[Table 10]**

| **Material name** | **Manufacturer** | **Model number** | **Use** | **Notes** |
|---|---|---|---|---|
| **Matrigel basement** | **Corning** | 356234 | | |
| **FluxPak assay pack** | **Agilent** | 103022-100 | **Mitochondrial test** | |
| Seahorse XF DMEM | **Agilent** | | **Mitochondrial test** | |
| Medium pH 7.4 | | | | |
| Seahorse XF Glucose (1.0 M solution) | **Agilent** | | **Mitochondrial test** | |
| Seahorse XF Pyruvate (100 mM solution) | **Agilent** | | **Mitochondrial test** | |
| Seahorse XF L-Glutamine (200 mM solution) | **Agilent** | | **Mitochondrial test** | |
| **Mitostress kit for XFp** | **Agilent** | 103010-100 | **Mitochondrial test** | |

The sensor cartridge attached to the XF HS mini (Agilent Technologies) was hydrated, and a dedicated plate was coated with Matrigel. The next day, the frozen human T cells (four lots) prepared in 1-1) on day 5 and day 10 were awakened, cultured for 1 hour, and then seeded onto the dedicated plate at 1x10^5/well. The cells were centrifuged at room temperature for 1 minute at 200xg. The analysis medium was added to the stock solutions of oligomycin, FCCP, and rotenone/antimycin A, and vortexed. The sensor cartridge was removed from the incubator, and oligomycin, FCCP, and rotenone/antimycin A were respectively added to ports A, B, and C. Using the XF HS mini, the mitochondrial spare respiratory capacity and ATP production rate of each human T cell were measured. The results are shown in Fig. 13 to Fig. 16. In Lot 2 and Lot 3, the total ATP production rate exceeded 400 pmol/min/10^5 cells on day 5 or day 6, and the mitochondrial spare respiratory capacity exceeded 40 pmol/min/10^5 cells. On the other hand, in Lot 1, the total ATP production rate exceeded 400 pmol/min/10^5 cells on day 5, but the mitochondrial spare respiratory capacity was below 40 pmol/min/10^5 cells. In Lot 4, both indicators were below the above-mentioned threshold values on day 5. The results are summarized in Table 11.

**[Table 11]**

| Lot No. of human T cell | total ATP production rate>400 | spare respiratory capacity>40 |
|---|---|---|
| Lot 1 | ○ | x |
| Lot 2 | ○ | ○ |
| Lot 3 | ○ | ○ |
| Lot 4 | x | x |

### 3) Correlation between proliferation ability of human T cells and ATP production rate and mitochondrial spare respiratory capacity

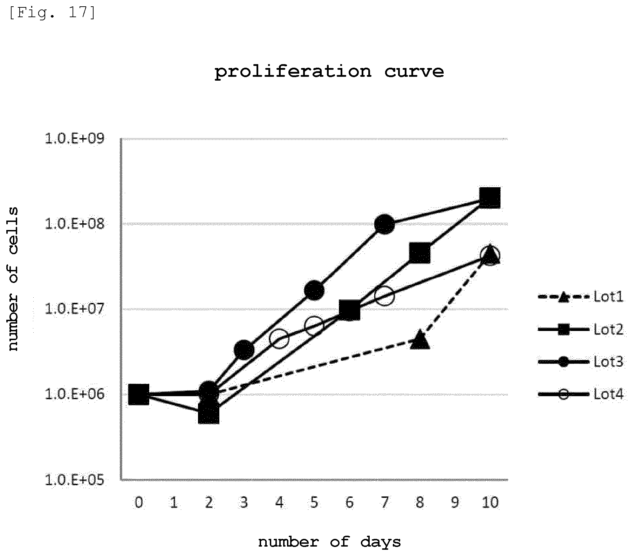

Each lot of human T cells was adjusted to 1×10^6/mL in KBM502 medium and expansion cultured until day 10. A portion was counted during the culture, and a proliferation curve was drawn. The results are shown in Fig. 17. Lot 2 and Lot 3 proliferated more than 100-fold by day 10, whereas Lot 1 and Lot 4 showed a slow proliferation rate.

### 4) Correlation between long-term maintenance ability of undifferentiated human T cells and ATP production rate and mitochondrial spare respiratory capacity

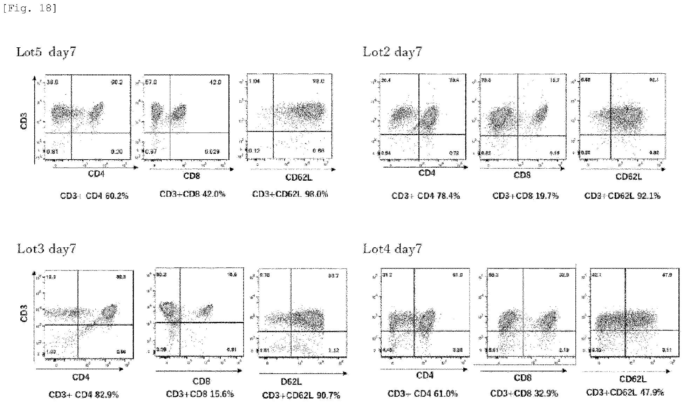

In the culture of 1-2), human T cells (Lot 2 to Lot 4) on day 7 were subjected to FACS analysis using various antibodies shown in Table 6, and the proportion of human CD4 positive cells, human CD8 positive cells, and human CD62L positive cells was examined. In addition, FACS analysis was also performed on another lot of human T cells (Lot 5) with a total ATP production rate of 400 pmol/min/10^5 cells or more and a mitochondrial spare respiratory capacity of 40 pmol/min/10^5 cells or more on day 5 or day 6. The results are shown in Fig. 18. In the three lots (Lot 2, Lot 3, Lot 5) in which both ATP production rate and mitochondrial spare respiratory capacity were above the threshold values, CD62L-positive human T cells accounted for 90% or more on day 7, but in Lot 4, in which both indices were below the threshold values, CD62L-positive human T cells decreased to less than 50% on day 7.

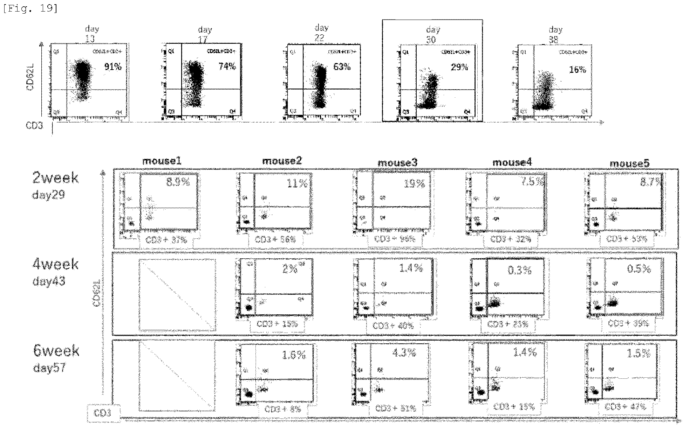

Furthermore, the proportion of CD3-positive CD62L-positive cells on days 13, 17, 22, 30, and 38 was measured and compared between Lot 3 and Lot 4. As a result, no CD3-positive CD62L-positive cells were detected in Lot 4, whereas 16% of Lot 3 were CD3-positive CD62L-positive cells even on day 38, indicating that undifferentiated human T cells were maintained (Fig. 19, upper panel).

### 5) Correlation between long-term retention ability of human T cells in recipients and ATP production rate and mitochondrial spare respiratory capacity

Using the materials shown in Table 12, transplantation test of human T cells into humanized mice was conducted.

**[Table 12]**

| **Material name** | **Manufacturer** | **Model number** | **Use** | **Notes** |
|---|---|---|---|---|
| NOD.Cg-PrkdcscidIl2rgtm1Sug/ShiJic | CIEA | | **animal transplantation test** | |
| BD Pharm Lyse^{™} Lysing Buffer | BD | | **animal transplantation test** | |

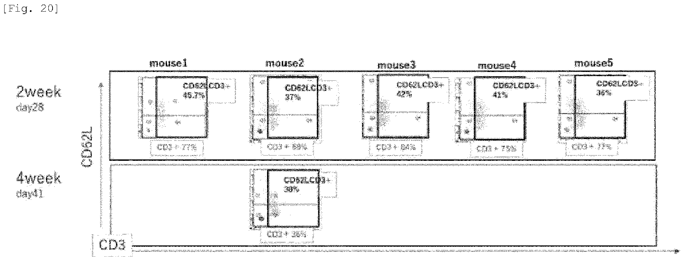

FACS was performed on the human T cells (Lot 3, Lot 4) obtained on day 10 in 1-2), by using the anti-CD3 and anti-CD62L antibodies shown in Table 6, and it was confirmed that the CD3-positive, CD62L-positive cell fraction was nearly 100%. 1x10^7 cells were suspended in 200 mL of PBS per NOG mouse and transplanted via tail vein injection (n=5). Blood was collected every 2 weeks, and after hemolysis, FACS analysis was performed using anti-CD3 antibody and anti-CD62L antibody. The results are shown in Fig. 19 and Fig. 20. In Lot 3, CD3-positive CD62L-positive cells remained in all four surviving mice even 6 weeks after transplantation (Fig. 19, lower panel), whereas in Lot 4, CD3-positive CD62L-positive cells were no longer detectable in four out of five mice 4 weeks after transplantation, and CD3-positive CD62L-positive cells disappeared in all mice 6 weeks after transplantation (Fig. 20).

### Example 6: Effect of frequency of medium addition on proliferation of human T cells

As shown in Table 13, two culture methods were performed by changing the interval of medium addition, and cell proliferation was compared.

**[Table 13]**

| Culture method 1 | | | Culture method 2 | |
|---|---|---|---|---|
| day | seeding concentration | operation | seeding concentration | operation |
| 0 | 2e6/mL | CD3/28 transact | 2e6/mL | CD3/28 transact |
| 2 | | | | medium addition |
| 3 | 5e5/mL | centrifuged to remove transact | 5e5/mL | centrifuged to remove transact |
| 5 | 5e5/mL | medium addition | 5e5/mL | medium addition |
| 6 | | | | medium addition |
| 7 | 5e5/mL | medium addition | 5e5/mL | medium addition |
| 10 | | cell collection | | cell collection |

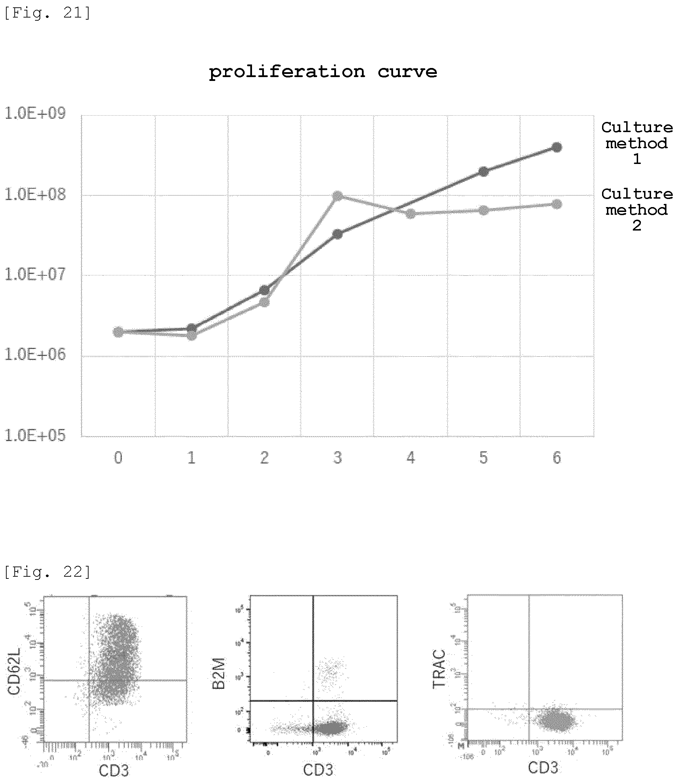

The results are shown in Fig. 21. It was shown that adding medium every 2-3 days resulted in higher proliferation efficiency of human T cells than adding medium every day.

### Example 7: Production of human T cells with B2M gene/TRAC gene double knockout and ST6GALNAC6 gene introduction

The B2M gene and TRAC gene were double knocked out and the ST6GALNAC6 gene was introduced into the human T cells of Example 3 in the same manner as in Example 4, except that the TRAC gene was knocked out along with the B2M gene in Fig. 7. Five days after introduction of sgRNA and Cas9, B2M and TRAC were confirmed to have been knocked out by FACS (Fig. 22). 97% of TRAC was knocked out. Then, the ST6GALNAC6 gene was introduced by lentivirus infection. After four days after infection, the cells were co-cultured with NK cells to confirm the establishment of immune tolerance.

### Experimental Example 3: Induction of immune tolerance to NK cell (3)

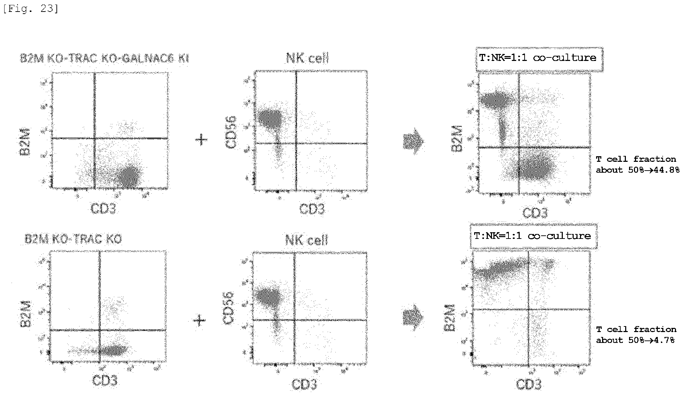

In the same manner as in Experimental Example 2, 1), human NK cells were isolated (Fig. 23, middle). Using the reagents and the like shown in Table 4, the genetically modified human T cells obtained in Example 7 (Fig. 23, upper left) and human NK cells were co-cultured at a ratio of T cell:NK cell = 1:1. For comparison, B2M KO/TRAC KO T cells (Fig. 23, lower left) without introduction of ST6GALNAC6 gene (mock) were also tested in the same manner. After counting the total cells, the CD3 percentage was confirmed by FACS, and changes in the ratio of T cell:NK cell were examined. As a result, it was confirmed that attacks from NK cells were highly suppressed in T cells introduced with the ST6GALNAC6 gene (Fig. 23, right).

While the present invention has been described with emphasis on preferred embodiments, it is obvious to those skilled in the art that the preferred embodiments can be modified.

The contents disclosed in any publication described herein, including patents and patent applications, are hereby incorporated in their entireties by reference, to the extent that they have been disclosed herein.

This application is based on a patent application No. 2022-145680 filed in Japan (filing date: September 13, 2022), the contents of which are incorporated in full herein by reference.

### [Industrial Applicability]

The human T cell of the present invention is extremely useful as a highly versatile human T cell that is not rejected by T cells or NK cells from the recipient side when allogeneically transplanted, does not attack non-target cells of the recipient, does not cause GVHD or has very low chance of causing GVHD, and can be used to produce a safe genetically modified T cell therapeutic agent. In addition, the human T cell of the present invention can survive stably in the body for a long period of time after transplantation, and therefore is also extremely useful in eradicating residual cancer cells that could not be eradicated by conventional T cell cancer immunotherapy, and improving the therapeutic effect.

## Claims

1. A human T cell lacking human leukocyte antigen (HLA) class I molecule and comprising an exogenous ST6GALNAC6 gene, which has the following characteristics (a) and/or (b):
(a) CD62L-positive and CD45RA-positive
(b) a total ATP production rate of a human T cell-containing cell population of 400 pmol/min/10⁵ cells or more and a mitochondrial spare respiratory capacity of 40 pmol/min/10⁵ cells or more, 5-6 days after the application of T cell proliferation stimulation to the cell population.

2. The human T cell according to claim 1, which is a naive T cell.

3. The human T cell according to claim 1 or 2, wherein the β2 microglobulin (B2M) gene is knocked out.

4. A method for producing a human T cell capable of sustained proliferation in vitro and/or long-term survival in a recipient after transplantation, comprising performing the following (a) or (b):
(a) selecting a CD62L-positive CD45RA-positive cell from a human T cell-containing cell population
(b) applying T cell proliferation stimulation to and culturing the test human T cell-containing cell population, measuring a total ATP production rate and a mitochondrial spare respiratory capacity of the cell population 5 - 6 days after the proliferation stimulation, and selecting a test cell population with a total ATP production rate of 400 pmol/min/10⁵ cells or more and a spare respiratory capacity of 40 pmol/min/10⁵ cells or more.

5. The method according to claim 4, comprising further expansion culturing human T cells selected by the aforementioned (a) or (b).

6. The method according to claim 4 or 5, wherein the culturing comprises adding a medium every 2 - 3 days.

7. A transplantation therapy agent comprising a human T cell having the following characteristics (a) and/or (b):
(a) CD62L-positive and CD45RA-positive
(b) a total ATP production rate of a human T cell-containing cell population of 400 pmol/min/10⁵ cells or more and a mitochondrial spare respiratory capacity of 40 pmol/min/10⁵ cells or more, 5-6 days after the application of T cell proliferation stimulation to the cell population.

8. The agent according to claim 7, wherein the human T cell is a low-immunogenic genetically engineered cell.

9. The agent according to claim 7 or 8, wherein the human T cell is a CAR-T cell or TCR-T cell.

10. A method for producing a low-immunogenic human T cell, comprising
(1) providing a human T cell having the following characteristics (a) and/or (b),
(a) CD62L-positive and CD45RA-positive
(b) a total ATP production rate of a human T cell-containing cell population of 400 pmol/min/10⁵ cells or more and a mitochondrial spare respiratory capacity of 40 pmol/min/10⁵ cells or more, 5-6 days after the application of T cell proliferation stimulation to the cell population
(2) deleting an HLA class I molecule in the human T cell or a progenitor cell thereof, and
(3) introducing exogeneous ST6GALNAC6 gene into the human T cell or a progenitor cell thereof.

11. The method according to claim 10, wherein the human T cell having the aforementioned characteristics (a) and/or (b) is a naive T cell.

12. The method according to claim 10 or 11, wherein the HLA class I molecule is deleted by knocking out the B2M gene.

13. A transplantation therapy agent comprising the human T cell according to any one of claims 1 to 3, or a human T cell obtained by the method according to any one of claims 10 to 12.

14. The agent according to claim 13, wherein the human T cell is a CAR-T cell or TCR-T cell.
